# EUROPEAN PATENT SPECIFICATION

(11) **EP 2 435 074 B1**
(45) Date of publication and mention of the grant of the patent: **25.11.2015**
(21) Application number: 10724012.9
(22) Date of filing: 26.05.2010
(51) Int. Cl.: A61K 39/12, A61K 39/145, C07K 14/11, G01N 33/53

(54) **EXTRANEOUS AGENTS TESTING**
TESTUNG VON FREMDSTOFFEN
TESTS D'AGENTS EXOGÈNES

(30) Priority: 28.05.2009 EP 09161368; 28.05.2009 US 181835 P
(43) Date of publication of application: 04.04.2012
(73) Proprietor: Abbott Biologicals B.V., 1381 CP Weesp (NL)
(72) Inventor: SCHOEN, Pieter Joseph, B-9620 Merelbeke (BE); KERSTEN, Alexander Jeroen, NL-1241 AP Kortenhoef (NL); MEDEMA, Jeroen Kristiaan, Abbott Park, IL 60064 (US); THUS, Johannes Lambertus Gerardus, NL-1381 CP Weesp (NL)
(74) Representative: Prüfer & Partner GbR European Patent Attorneys
(86) International application number: PCT/EP2010/057221
(87) International publication number: WO 2010/136476

(56) References cited:
- EP-A- 0 236 145
- EP-A- 1 997 831
- WO-A-03/072811
- WO-A-2007/100397
- WO-A-2008/124331
- US-A1- 2004 053 388
- US-A1- 2006 153 872
- GREGERSEN ET AL: "A risk-assessment model to rate the occurrence and relevance of adventitious agents in the production of influenza vaccines" VACCINE, BUTTERWORTH SCIENTIFIC. GUILDFORD, GB, vol. 26, no. 26, 19 June 2008 (2008-06-19) , pages 3297-3304, XP022710580 ISSN: 0264-410X [retrieved on 2008-04-15]
- QUINTANA ET AL: "Detection of porcine circovirus type 1 in commercial pig vaccines using polymerase chain reaction" VETERINARY JOURNAL, BAILLIERE TINDALL, LONDON, GB, vol. 171, no. 3, 1 May 2006 (2006-05-01), pages 570-573, XP005368534 ISSN: 1090-0233
- HALDER M: "Three Rs potential in the development and quality control of immunobiologicals." ALTEX : ALTERNATIVEN ZU TIEREXPERIMENTEN 2001, vol. 18 Suppl 1, 2001, pages 13-47, XP009126466 ISSN: 0946-7785

### SEQUENCE LISTING

<110> Solvay Pharmaceuticals B.V.
<120> Extraneous agents testing
<130> WK 1280
<160> 4
<170> PatentIn version 3.3
<210> 1
   <211> 1698
   <212> DNA
   <213> Artificial
<220>
   <223> codon optimized hemagglutinin (HA) antigen
<400> 1
   atggagaaaa ttgtgctgct gttcgccatt gtgtccctgg tgaagtccga ccagatctgc      60
   atcggctatc acgccaacaa ctccaccgag caggtcgaca ccattatgga aaagaatgtg     120
   accgtgaccc atgcacaaga catcctggaa aagacccaca acggcaaact gtgcgacctg     180
   gacggcgtga agcccctgat cctgcgcgac tgctccgtgg ctgggtggct gctgggaaac     240
   cctatgtgcg acgagttcat caatgtgccc gagtggtcct acatcgttga gaaagccaac     300
   cccgtcaacg acctgtgtta ccccggtgac ttcaacgact acgaggagct caagcacctg     360
   ctctcccgga tcaaccactt cgagaaaatc cagatcatcc ccaaatcctc ctggtccagc     420
   cacgaagcct ccctcggcgt gtcctccgct tgtccctacc agggcaagtc ctccttcttc     480
   cgcaacgtcg tgtggctgat caaaaagaac tccacctacc ccaccattaa gcggtcctac     540
   aacaacacca accaggaaga cctgctggtg ctgtggggca tccaccatcc caacgacgcc     600
   gctgagcaga ccaagctgta ccagaaccct accacctaca tctccgtcgg gacatccaca     660
   ctgaaccagc gcctcgtgcc ccgcattgcc acccgcagca aggtgaatgg gcagtccggc     720
   cgcatggagt tcttctggac aatcctgaag cccaacgacg ctatcaactt cgagtccaac     780
   ggcaacttca tcgctcccga gtatgcctac aagatcgtta aaaagggcga ctccacaatc     840
   atgaagtccg agttggagta cggcaactgc aacaccaagt gccagacacc catgggcgct     900
   atcaactcct ccatgccctt ccacaacatc catcccctga ccattggcga gtgccccaag     960
   tacgtcaagt ccaaccggct ggtcctggct accggcctcc gcaactcccc tcaaagggaa    1020
   cgcaggcgca aaaagagagg cctgttcgga gccatagctg ggttcattga gggagggtgg    1080
   cagggcatgg tggatgggtg gtacggctac catcactcca acgagcaggg ctccgggtat    1140
   gccgccgaca aggagtccac ccagaaggcc attgacggcg tgaccaacaa ggtgaacagc    1200
   atcatcgaca agatgaacac ccagttcgag gctgttggcc gggagttcaa caacttggag    1260
   cgccgcatcg agaacctgaa caaaaagatg gaggacggct tcctggatgt gtggacctac    1320
   aacgctgagc tcctggtgct gatggagaac gagcggaccc tggacttcca cgactccaac    1380
   gtcaagaacc tgtacgacaa agtgcgcctc cagctgcggg acaacgccaa ggagctcggg    1440
   aatggctgct tcgagttcta ccacaagtgc gacaacgagt gtatggagtc cgtgcgcaat    1500
   ggcacctacg actatcccca gtactccgaa gaggcccgcc tgaagcgcga agagatctcc    1560
   ggggtgaagt tggagtccat tggcatctac cagatcctga gcatctactc caccgtggct    1620
   tcctccctcg ccctcgccat tatggtggct ggcctgtccc tgtggatgtg ctccaacggc    1680
   tccctgcaat gtcgctaa                                                  1698
<210> 2
   <211> 1350
   <212> DNA
   <213> Artificial
<220>
   <223> codon optimized neuraminidase (NA) antigen
<400> 2
   atgaacccca accagaagat catcaccatt ggcagcatct gcatggtgac cgggatcgtt      60
   agcctgatgc tgcagatcgg caacatgatc agcatctggg tgtcccacag catccatacc     120
   ggcaaccagc accagtccga gcccatctcc aacaccaacc tgctgaccga gaaagctgtc     180
   gcctccgtga agctggctgg caactcctcc ctgtgtccta tcaatgggtg ggctgtgtac     240
   tccaaggaca actccattcg catcggcagc aaaggggatg tcttcgtgat ccgcgagcct     300
   ttcatctcct gtagccacct cgaatgccgg accttcttcc tgacacaagg agccctgctg     360
   aacgacaagc actccaacgg caccgtgaag gaccgctctc cccaccgcac cctgatgtcc     420
   tgccccgtcg gcgaagcccc tagcccctac aactcccggt tcgagtccgt cgcctggagc     480
   gcctctgcct gtcatgatgg gacctcttgg ctgaccattg gcatctccgg ccctgataat     540
   ggagcagtgg ctgtgctgaa gtacaacggc atcatcaccg ataccattaa gtcctggcgc     600
   aacaacatcc tccgcaccca ggagtccgag tgtgcctgtg tcaatggctc gtgctttacc     660
   gtgatgaccg atgggccaag caatggacaa gcctcccaca agatcttcaa gatggaaaag     720
   ggcaaggtgg tgaagtccgt cgagttggac gcccctaatt accactacga ggagtgcagc     780
   tgttaccctg acgctgggga gatcacctgt gtgtgcaggg acaactggca cgggagcaac     840
   aggccttggg tctccttcaa ccagaacttg gagtaccaga tcggctacat ctgttccggg     900
   gtgttcgggg ataatcccag gcctaacgat ggcaccgggt cctgtggccc tgtgagctcc     960
   aacggcgcag gcggggtcaa gggcttctcg ttcaaatatg gcaatggcgt gtggatcggg    1020
   cgcaccaagt ccacaaactc ccggtccggc ttcgagatga tctgggaccc caatggatgg    1080
   accgagacag acagctcctt ctccgtcaag caggacattg tggctatcac cgactggagc    1140
   gggtacagcg gcagcttcgt gcagcacccc gagctcacag gcctcgactg cattcggccc    1200
   tgcttttggg tggaactgat ccggggacgg cctaaggaga gcacaatctg gacctccggc    1260
   tccagcatct ccttctgcgg cgtgaactcc gacacagtgg ggtggtcctg gcctgacgga    1320
   gctgaactgc ccttcaccat tgacaagtaa                                     1350
<210> 3
   <211> 5687
   <212> DNA
   <213> Artificial
<220>
   <223> whole plasmid DNA sequences of pCMV-HA
<400> 3
   tcaatattgg ccattagcca tattattcat tggttatata gcataaatca atattggcta      60
   ttggccattg catacgttgt atctatatca taatatgtac atttatattg gctcatgtcc     120
   aatatgaccg ccatgttggc attgattatt gactagttat taatagtaat caattacggg     180
   gtcattagtt catagcccat atatggagtt ccgcgttaca taacttacgg taaatggccc     240
   gcctggctga ccgcccaacg acccccgccc attgacgtca ataatgacgt atgttcccat     300
   agtaacgcca atagggactt tccattgacg tcaatgggtg gagtatttac ggtaaactgc     360
   ccacttggca gtacatcaag tgtatcatat gccaagtccg ccccctattg acgtcaatga     420
   cggtaaatgg cccgcctggc attatgccca gtacatgacc ttacgggact ttcctacttg     480
   gcagtacatc tacgtattag tcatcgctat taccatggtg atgcggtttt ggcagtacac     540
   caatgggcgt ggatagcggt ttgactcacg gggatttcca agtctccacc ccattgacgt     600
   caatgggagt ttgttttggc accaaaatca acgggacttt ccaaaatgtc gtaataaccc     660
   cgccccgttg acgcaaatgg gcggtaggcg tgtacggtgg gaggtctata taagcagagc     720
   tcgtttagtg aaccgtcaga tcactagaag ctttattgcg gtagtttatc acagttaaat     780
   tgctaacgca gtcagtgctt ctgacacaac agtctcgaac ttaagctgca gaagttggtc     840
   gtgaggcact gggcaggtaa gtatcaaggt tacaagacag gtttaaggag accaatagaa     900
   actgggcttg tcgagacaga gaagactctt gcgtttctga taggcaccta ttggtcttac     960
   tgacatccac tttgcctttc tctccacagg tgtccactcc cagttcaatt acagctctta    1020
   aggctagagt acttaatacg actcactata ggctagcgcc accatggaga aaattgtgct    1080
   gctgttcgcc attgtgtccc tggtgaagtc cgaccagatc tgcatcggct atcacgccaa    1140
   caactccacc gagcaggtcg acaccattat ggaaaagaat gtgaccgtga cccatgcaca    1200
   agacatcctg gaaaagaccc acaacggcaa actgtgcgac ctggacggcg tgaagcccct    1260
   gatcctgcgc gactgctccg tggctgggtg gctgctggga aaccctatgt gcgacgagtt    1320
   catcaatgtg cccgagtggt cctacatcgt tgagaaagcc aaccccgtca acgacctgtg    1380
   ttaccccggt gacttcaacg actacgagga gctcaagcac ctgctctccc ggatcaacca    1440
   cttcgagaaa atccagatca tccccaaatc ctcctggtcc agccacgaag cctccctcgg    1500
   cgtgtcctcc gcttgtccct accagggcaa gtcctccttc ttccgcaacg tcgtgtggct 1560
   gatcaaaaag aactccacct accccaccat taagcggtcc tacaacaaca ccaaccagga 1620
   agacctgctg gtgctgtggg gcatccacca tcccaacgac gccgctgagc agaccaagct 1680
   gtaccagaac cctaccacct acatctccgt cgggacatcc acactgaacc agcgcctcgt 1740
   gccccgcatt gccacccgca gcaaggtgaa tgggcagtcc ggccgcatgg agttcttctg 1800
   gacaatcctg aagcccaacg acgctatcaa cttcgagtcc aacggcaact tcatcgctcc 1860
   cgagtatgcc tacaagatcg ttaaaaaggg cgactccaca atcatgaagt ccgagttgga 1920
   gtacggcaac tgcaacacca agtgccagac acccatgggc gctatcaact cctccatgcc 1980
   cttccacaac atccatcccc tgaccattgg cgagtgcccc aagtacgtca agtccaaccg 2040
   gctggtcctg gctaccggcc tccgcaactc ccctcaaagg gaacgcaggc gcaaaaagag 2100
   aggcctgttc ggagccatag ctgggttcat tgagggaggg tggcagggca tggtggatgg 2160
   gtggtacggc taccatcact ccaacgagca gggctccggg tatgccgccg acaaggagtc 2220
   cacccagaag gccattgacg gcgtgaccaa caaggtgaac agcatcatcg acaagatgaa 2280
   cacccagttc gaggctgttg gccgggagtt caacaacttg gagcgccgca tcgagaacct 2340
   gaacaaaaag atggaggacg gcttcctgga tgtgtggacc tacaacgctg agctcctggt 2400
   gctgatggag aacgagcgga ccctggactt ccacgactcc aacgtcaaga acctgtacga 2460
   caaagtgcgc ctccagctgc gggacaacgc caaggagctc gggaatggct gcttcgagtt 2520
   ctaccacaag tgcgacaacg agtgtatgga gtccgtgcgc aatggcacct acgactatcc 2580
   ccagtactcc gaagaggccc gcctgaagcg cgaagagatc tccggggtga agttggagtc 2640
   cattggcatc taccagatcc tgagcatcta ctccaccgtg gcttcctccc tcgccctcgc 2700
   cattatggtg gctggcctgt ccctgtggat gtgctccaac ggctccctgc aatgtcgcta 2760
   atctagagtc gacctgggcg gccgcttcga gcagacatga taagatacat tgatgagttt 2820
   ggacaaacca caactagaat gcagtgaaaa aaatgcttta tttgtgaaat ttgtgatgct 2880
   attgctttat ttgtaaccat tataagctgc aataaacaag ttaacaacaa caattgcatt 2940
   cattttatgt ttcaggttca gggggagatg tgggaggttt tttaaagcaa gtaaaacctc 3000
   tacaaatgtg gtaaaatcga taagtatccg ggctggcgta atagcgaaga ggcccgcacc 3060
   gatcgccctt cccaacagtt gcgcagcctg aatggcgaat ggacgcgccc tgtagcggcg 3120
   cattaagcgc ggcgggtgtg gtggttacgc gcagcgtgac cgctacactt gccagcgccc 3180
   tagcgcccgc tcctttcgct ttcttccctt cctttctcgc cacgttcgcc ggctttcccc 3240
   gtcaagctct aaatcggggg ctccctttag ggttccgatt tagtgcttta cggcacctcg 3300
   accccaaaaa acttgattag ggtgatggtt cacgtagtgg gccatcgccc tgatagacgg 3360
   tttttcgccc tttgacgttg gagtccacgt tctttaatag tggactcttg ttccaaactg 3420
   gaacaacact caaccctatc tcggtctatt cttttgattt ataagggatt ttgccgattt 3480
   cggcctattg gttaaaaaat gagctgattt aacaaaaatt taacgcgaat tttaacaaaa 3540
   tattaacgct tacaatttcc tgatgcggta ttttctcctt acgcatctgt gcggtatttc 3600
   acaccgcata tggtgcactc tcagtacaat ctgctctgat gccgcatagt taagccagcc 3660
   ccgacacccg ccaacacccg ctgacgcgcc ctgacgggct tgtctgctcc cggcatccgc 3720
   ttacagacaa gctgtgaccg tctccgggag ctgcatgtgt cagaggtttt caccgtcatc 3780
   accgaaacgc gcgagacgaa agggcctcgt gatacgccta tttttatagg ttaatgtcat 3840
   gataataatg gtttcttaga cgtcaggtgg cacttttcgg ggaaatgtgc gcggaacccc 3900
   tatttgttta tttttctaaa tacattcaaa tatgtatccg ctcatgagac aataaccctg 3960
   ataaatgctt caataatatt gaaaaaggaa gagtatgagt attcaacatt tccgtgtcgc 4020
   ccttattccc ttttttgcgg cattttgcct tcctgttttt gctcacccag aaacgctggt 4080
   gaaagtaaaa gatgctgaag atcagttggg tgcacgagtg ggttacatcg aactggatct 4140
   caacagcggt aagatccttg agagttttcg ccccgaagaa cgttttccaa tgatgagcac 4200
   ttttaaagtt ctgctatgtg gcgcggtatt atcccgtatt gacgccgggc aagagcaact 4260
   cggtcgccgc atacactatt ctcagaatga cttggttgag tactcaccag tcacagaaaa 4320
   gcatcttacg gatggcatga cagtaagaga attatgcagt gctgccataa ccatgagtga 4380
   taacactgcg gccaacttac ttctgacaac gatcggagga ccgaaggagc taaccgcttt 4440
   tttgcacaac atgggggatc atgtaactcg ccttgatcgt tgggaaccgg agctgaatga 4500
   agccatacca aacgacgagc gtgacaccac gatgcctgta gcaatggcaa caacgttgcg 4560
   caaactatta actggcgaac tacttactct agcttcccgg caacaattaa tagactggat 4620
   ggaggcggat aaagttgcag gaccacttct gcgctcggcc cttccggctg gctggtttat 4680
   tgctgataaa tctggagccg gtgagcgtgg gtctcgcggt atcattgcag cactggggcc 4740
   agatggtaag ccctcccgta tcgtagttat ctacacgacg gggagtcagg caactatgga 4800
   tgaacgaaat agacagatcg ctgagatagg tgcctcactg attaagcatt ggtaactgtc 4860
   agaccaagtt tactcatata tactttagat tgatttaaaa cttcattttt aatttaaaag 4920
   gatctaggtg aagatccttt ttgataatct catgaccaaa atcccttaac gtgagttttc 4980
   gttccactga gcgtcagacc ccgtagaaaa gatcaaagga tcttcttgag atcctttttt 5040
   tctgcgcgta atctgctgct tgcaaacaaa aaaaccaccg ctaccagcgg tggtttgttt 5100
   gccggatcaa gagctaccaa ctctttttcc gaaggtaact ggcttcagca gagcgcagat 5160
   accaaatact gttcttctag tgtagccgta gttaggccac cacttcaaga actctgtagc 5220
   accgcctaca tacctcgctc tgctaatcct gttaccagtg gctgctgcca gtggcgataa 5280
   gtcgtgtctt accgggttgg actcaagacg atagttaccg gataaggcgc agcggtcggg 5340
   ctgaacgggg ggttcgtgca cacagcccag cttggagcga acgacctaca ccgaactgag 5400
   atacctacag cgtgagctat gagaaagcgc cacgcttccc gaagggagaa aggcggacag 5460
   gtatccggta agcggcaggg tcggaacagg agagcgcacg agggagcttc cagggggaaa 5520
   cgcctggtat ctttatagtc ctgtcgggtt tcgccacctc tgacttgagc gtcgattttt 5580
   gtgatgctcg tcaggggggc ggagcctatg gaaaaacgcc agcaacgcgg cctttttacg 5640
   gttcctggcc ttttgctggc cttttgctca catggctcga cagatct 5687
<210> 4
   <211> 5339
   <212> DNA
   <213> Artificial
<220>
   <223> whole plasmid DNA sequences of pCMV-NA
<400> 4
   tcaatattgg ccattagcca tattattcat tggttatata gcataaatca atattggcta 60
   ttggccattg catacgttgt atctatatca taatatgtac atttatattg gctcatgtcc 120
   aatatgaccg ccatgttggc attgattatt gactagttat taatagtaat caattacggg 180
   gtcattagtt catagcccat atatggagtt ccgcgttaca taacttacgg taaatggccc 240
   gcctggctga ccgcccaacg acccccgccc attgacgtca ataatgacgt atgttcccat 300
   agtaacgcca atagggactt tccattgacg tcaatgggtg gagtatttac ggtaaactgc 360
   ccacttggca gtacatcaag tgtatcatat gccaagtccg ccccctattg acgtcaatga 420
   cggtaaatgg cccgcctggc attatgccca gtacatgacc ttacgggact ttcctacttg 480
   gcagtacatc tacgtattag tcatcgctat taccatggtg atgcggtttt ggcagtacac 540
   caatgggcgt ggatagcggt ttgactcacg gggatttcca agtctccacc ccattgacgt 600
   caatgggagt ttgttttggc accaaaatca acgggacttt ccaaaatgtc gtaataaccc 660
   cgccccgttg acgcaaatgg gcggtaggcg tgtacggtgg gaggtctata taagcagagc 720
   tcgtttagtg aaccgtcaga tcactagaag ctttattgcg gtagtttatc acagttaaat 780
   tgctaacgca gtcagtgctt ctgacacaac agtctcgaac ttaagctgca gaagttggtc 840
   gtgaggcact gggcaggtaa gtatcaaggt tacaagacag gtttaaggag accaatagaa 900
   actgggcttg tcgagacaga gaagactctt gcgtttctga taggcaccta ttggtcttac 960
   tgacatccac tttgcctttc tctccacagg tgtccactcc cagttcaatt acagctctta 1020
   aggctagagt acttaatacg actcactata ggctagcgcc accatgaacc ccaaccagaa 1080
   gatcatcacc attggcagca tctgcatggt gaccgggatc gttagcctga tgctgcagat 1140
   cggcaacatg atcagcatct gggtgtccca cagcatccat accggcaacc agcaccagtc 1200
   cgagcccatc tccaacacca acctgctgac cgagaaagct gtcgcctccg tgaagctggc 1260
   tggcaactcc tccctgtgtc ctatcaatgg gtgggctgtg tactccaagg acaactccat 1320
   tcgcatcggc agcaaagggg atgtcttcgt gatccgcgag cctttcatct cctgtagcca 1380
   cctcgaatgc cggaccttct tcctgacaca aggagccctg ctgaacgaca agcactccaa 1440
   cggcaccgtg aaggaccgct ctccccaccg caccctgatg tcctgccccg tcggcgaagc 1500
   ccctagcccc tacaactccc ggttcgagtc cgtcgcctgg agcgcctctg cctgtcatga 1560
   tgggacctct tggctgacca ttggcatctc cggccctgat aatggagcag tggctgtgct 1620
   gaagtacaac ggcatcatca ccgataccat taagtcctgg cgcaacaaca tcctccgcac 1680
   ccaggagtcc gagtgtgcct gtgtcaatgg ctcgtgcttt accgtgatga ccgatgggcc 1740
   aagcaatgga caagcctccc acaagatctt caagatggaa aagggcaagg tggtgaagtc 1800
   cgtcgagttg gacgccccta attaccacta cgaggagtgc agctgttacc ctgacgctgg 1860
   ggagatcacc tgtgtgtgca gggacaactg gcacgggagc aacaggcctt gggtctcctt 1920
   caaccagaac ttggagtacc agatcggcta catctgttcc ggggtgttcg gggataatcc 1980
   caggcctaac gatggcaccg ggtcctgtgg ccctgtgagc tccaacggcg caggcggggt 2040
   caagggcttc tcgttcaaat atggcaatgg cgtgtggatc gggcgcacca agtccacaaa 2100
   ctcccggtcc ggcttcgaga tgatctggga ccccaatgga tggaccgaga cagacagctc 2160
   cttctccgtc aagcaggaca ttgtggctat caccgactgg agcgggtaca gcggcagctt 2220
   cgtgcagcac cccgagctca caggcctcga ctgcattcgg ccctgctttt gggtggaact 2280
   gatccgggga cggcctaagg agagcacaat ctggacctcc ggctccagca tctccttctg 2340
   cggcgtgaac tccgacacag tggggtggtc ctggcctgac ggagctgaac tgcccttcac 2400
   cattgacaag taatctagag tcgacctggg cggccgcttc gagcagacat gataagatac 2460
   attgatgagt ttggacaaac cacaactaga atgcagtgaa aaaaatgctt tatttgtgaa 2520
   atttgtgatg ctattgcttt atttgtaacc attataagct gcaataaaca agttaacaac 2580
   aacaattgca ttcattttat gtttcaggtt cagggggaga tgtgggaggt tttttaaagc 2640
   aagtaaaacc tctacaaatg tggtaaaatc gataagtatc cgggctggcg taatagcgaa 2700
   gaggcccgca ccgatcgccc ttcccaacag ttgcgcagcc tgaatggcga atggacgcgc 2760
   cctgtagcgg cgcattaagc gcggcgggtg tggtggttac gcgcagcgtg accgctacac 2820
   ttgccagcgc cctagcgccc gctcctttcg ctttcttccc ttcctttctc gccacgttcg 2880
   ccggctttcc ccgtcaagct ctaaatcggg ggctcccttt agggttccga tttagtgctt 2940
   tacggcacct cgaccccaaa aaacttgatt agggtgatgg ttcacgtagt gggccatcgc 3000
   cctgatagac ggtttttcgc cctttgacgt tggagtccac gttctttaat agtggactct 3060
   tgttccaaac tggaacaaca ctcaacccta tctcggtcta ttcttttgat ttataaggga 3120
   ttttgccgat ttcggcctat tggttaaaaa atgagctgat ttaacaaaaa tttaacgcga 3180
   attttaacaa aatattaacg cttacaattt cctgatgcgg tattttctcc ttacgcatct 3240
   gtgcggtatt tcacaccgca tatggtgcac tctcagtaca atctgctctg atgccgcata 3300
   gttaagccag ccccgacacc cgccaacacc cgctgacgcg ccctgacggg cttgtctgct 3360
   cccggcatcc gcttacagac aagctgtgac cgtctccggg agctgcatgt gtcagaggtt 3420
   ttcaccgtca tcaccgaaac gcgcgagacg aaagggcctc gtgatacgcc tatttttata 3480
   ggttaatgtc atgataataa tggtttctta gacgtcaggt ggcacttttc ggggaaatgt 3540
   gcgcggaacc cctatttgtt tatttttcta aatacattca aatatgtatc cgctcatgag 3600
   acaataaccc tgataaatgc ttcaataata ttgaaaaagg aagagtatga gtattcaaca 3660
   tttccgtgtc gcccttattc ccttttttgc ggcattttgc cttcctgttt ttgctcaccc 3720
   agaaacgctg gtgaaagtaa aagatgctga agatcagttg ggtgcacgag tgggttacat 3780
   cgaactggat ctcaacagcg gtaagatcct tgagagtttt cgccccgaag aacgttttcc 3840
   aatgatgagc acttttaaag ttctgctatg tggcgcggta ttatcccgta ttgacgccgg 3900
   gcaagagcaa ctcggtcgcc gcatacacta ttctcagaat gacttggttg agtactcacc 3960
   agtcacagaa aagcatctta cggatggcat gacagtaaga gaattatgca gtgctgccat 4020
   aaccatgagt gataacactg cggccaactt acttctgaca acgatcggag gaccgaagga 4080
   gctaaccgct tttttgcaca acatggggga tcatgtaact cgccttgatc gttgggaacc 4140
   ggagctgaat gaagccatac caaacgacga gcgtgacacc acgatgcctg tagcaatggc 4200
   aacaacgttg cgcaaactat taactggcga actacttact ctagcttccc ggcaacaatt 4260
   aatagactgg atggaggcgg ataaagttgc aggaccactt ctgcgctcgg cccttccggc 4320
   tggctggttt attgctgata aatctggagc cggtgagcgt gggtctcgcg gtatcattgc 4380
   agcactgggg ccagatggta agccctcccg tatcgtagtt atctacacga cggggagtca 4440
   ggcaactatg gatgaacgaa atagacagat cgctgagata ggtgcctcac tgattaagca 4500
   ttggtaactg tcagaccaag tttactcata tatactttag attgatttaa aacttcattt 4560
   ttaatttaaa aggatctagg tgaagatcct ttttgataat ctcatgacca aaatccctta 4620
   acgtgagttt tcgttccact gagcgtcaga ccccgtagaa aagatcaaag gatcttcttg 4680
   agatcctttt tttctgcgcg taatctgctg cttgcaaaca aaaaaaccac cgctaccagc 4740
   ggtggtttgt ttgccggatc aagagctacc aactcttttt ccgaaggtaa ctggcttcag 4800
   cagagcgcag ataccaaata ctgttcttct agtgtagccg tagttaggcc accacttcaa 4860
   gaactctgta gcaccgccta catacctcgc tctgctaatc ctgttaccag tggctgctgc 4920
   cagtggcgat aagtcgtgtc ttaccgggtt ggactcaaga cgatagttac cggataaggc 4980
   gcagcggtcg ggctgaacgg ggggttcgtg cacacagccc agcttggagc gaacgaccta 5040
   caccgaactg agatacctac agcgtgagct atgagaaagc gccacgcttc ccgaagggag 5100
   aaaggcggac aggtatccgg taagcggcag ggtcggaaca ggagagcgca cgagggagct 5160
   tccaggggga aacgcctggt atctttatag tcctgtcggg tttcgccacc tctgacttga 5220
   gcgtcgattt ttgtgatgct cgtcaggggg gcggagccta tggaaaaacg ccagcaacgc 5280
   ggccttttta cggttcctgg ccttttgctg gccttttgct cacatggctc gacagatct    5339

## Description

### Field of the invention

The present invention belongs to the field of pharmaceutical industry and relates to a method for testing extraneous agents in a composition comprising at least one active agent and to a process for producing a pharmaceutical composition by carrying out said method. Furthermore, the present invention also relates to the use of a polynucleotide construct for testing the presence or absence of the active agent or of any extraneous or infectious agent in a composition to be tested. Moreover, it relates to polynucleotides, polynucleotide constructs comprising particular polynucleotides and host cells comprising these polynucleotides or polynucleotide constructs useful for the field of virus antigen preparations, notably relating to influenza virus. Also provided are non-human organisms, transgenic animals or microorganisms containing such polynucleotides and/or polynucleotide constructs. Furthermore, it relates to an antibody specific for a polypeptide encoded by said polynucleotide and to a method for producing said antibody, as well as to the use of an antibody raised against an expression product of a polynucleotide construct for the purification of an active agent. The present invention is also directed to kit of parts.

### Description of the background art

In the field of pharmaceutical industry there is the demand to produce compositions that are free of contaminants such as extraneous or adventitious agents, as these may result in undesired side effects. This demand is particularly challenging in the vaccine field. Absence of extraneous or adventitious agents may also be a regulatory issue.

To ensure that the compositions do not contain contaminations, the respective compositions can be tested whether they contain such contaminations or not.

In general, such tests can for instance be based on the detection of a possible extraneous agent being present in a composition, optionally with a prior amplification of the extraneous agent.

In this respect, WO 0172964 A2 describes a quantitative PCR method for the simultaneous detection and quantification of a viable adventitious agent in a sample of biologically-derived materials. This method comprises the measuring of the amount of a polynucleotide in a sample using quantitative polymerase chain reaction (PCR), incubating the sample under conditions that allow for the replication of the agent, measuring the amount of the polynucleotide in the sample after an incubation period using a quantitative PCR and comparing the amount of the polynucleotide present in the sample before and after the incubation period. An increase in the amount of polynucleotide indicates the presence of a viable agent in the sample.

WO 2007/100397 A2 refers to the identification or determination of the presence or absence of an adventitious contaminant virus in a sample comprising contacting nucleic acids from the sample with at least one primer pair, and determining the molecular mass of the amplification product by mass spectrometry.

Another possibility is to neutralize the active agent being present in the composition to be tested prior to adventitious agent testing. After the neutralization of the active agent, the neutralized composition is added to a specific cell line or is tested in animal models. If the cell line exhibits pathogenic effects, the composition contains adventitious agents.

In this context, US 2004/0005546 A1 provides a method for the detection of adventitious agents in a composition comprising a reovirus by using a ribozyme that specifically cleaves the genome of the reovirus, thereby inactivating the virus. A plasmid encoding this ribozyme is introduced in cells that are susceptible to reovirus infection. The transfected cells, by expressing the ribozyme, are capable of inactivating the reovirus and thus will not be infected by the virus. The ribozyme-expressing cells are then subjected to a composition containing reovirus, and any pathogenic effects caused by the reovirus preparation will indicate the presence of an adventitious agent. WO 03072811 A2 essentially refers to the same principle.

The neutralization of the active agent in the composition may also be achieved by the use of an antibody specific for the active agent, as it is described e.g. in the European Pharmacopoeia, chapter 2.6.16.. For instance if the active agent is a vaccine virus antigen, this vaccine virus antigen can be neutralized by antiserum containing antibodies specifically binding to the antigen. To prepare antiserum, an immunizing antigen produced in cell culture or other system (e.g. embryonated hens' eggs) from a species different from that used for the production of the vaccine and free from extraneous agents is used.

EP 0 236 145 A refers to processes for the production of HCMV glycoproteins, antibodies thereto and HCMV vaccines, and recombinant vectors therefor.

EP 1 997 831 A1 refers to chimeric vaccine antigens against Avian Influenza virus (Al). The chimeric antigens disclosed in EP 1 997 831 A1 comprise the virus subunits of Avian Influenza virus, coupled to co-stimulatory molecules that enhance both the humoral and cellular immune system.

WO 2008/124331 A1 defines novel sequences designed to protect a host against a virulent strain of H5N1 influenza A virus and discloses codon-optimized sequences encoding for haemagglutinin and neuraminidase antigenic viral proteins.

However, despite the above described methods for carrying out extraneous agent testing, there is still a need for an improved testing method, in particular for a non-specific testing method for possible identification of nonspecified infectious contaminants and for useful tools to perform suitable tests, and consequently for an improved production system for pharmaceutical compositions, in particular in the vaccine field.

### Summary of the invention

The present invention provides a subject-matter as defined in the appended claims.

The step of determining the presence or absence of extraneous agents in the composition may comprise:
a) using a non-human animal that has been inoculated, preferably immunized, with a polynucleotide construct comprising a sequence encoding at least a part of the active agent, and inoculating said animal with the composition to be tested,
b) assessing the percentage of living animals after a certain period of time, wherein in case at least 80% of the inoculated animals survived and did not show evidence of infection during said time period the composition is regarded as not containing extraneous agents, and in case less than 80% of the inoculated animals survived and/or at least one animal showed evidence of infection during said time period the composition is regarded as containing extraneous agents.

Further, the step of determining the presence or absence of extraneous agents in the composition may comprise:
a) inoculating a non-human animal with the composition to be tested, containing neutralized or inactivated active agent,
b) assessing the percentage of living animals after a certain period of time, wherein in case at least 80% of the inoculated animals survived and did not show evidence of infection during said time period the composition is regarded as not containing extraneous agents, and in case less than 80% of the inoculated animals survived and/or at least one animal showed evidence of infection during said time period the composition is regarded as containing extraneous agents.

The step of determining the presence or absence of extraneous agents in the composition to be tested can for instance be carried out by two ways: One possibility is to neutralize the active agent that is present in the composition to be tested prior to inoculating a test organism of a non-human animal, with the composition. This neutralization is carried out *in vitro,* i.e. not in the test organism itself. The test organism is inoculated with the composition to be tested after the composition has been neutralized. The other possibility is that the neutralization step is carried out *in situ*, e.g. in the test organism itself. This is achieved by an active immunization of the test organism with the polynucleotide construct comprising a sequence encoding at least a part of the active agent. By doing so, the test organism raises antibodies being directed against at least a part of the active agent. As soon as the test organism is inoculated with the composition to be tested (and that contains active agent not yet neutralized), these antibodies, in turn, are able to neutralize the active agent. Therefore, the determination of the presence or absence of extraneous agents in the composition to be tested can be carried out in the same test organism without any need for an in vitro neutralization step of the active agent. This significantly reduces the time needed for carrying out the testing method, the number of test organisms needed and further increases the robustness and safety of the test.

The non-human animals may be selected from the group consisting of adult mice, suckling mice, and guinea pigs, and wherein the percentage of living animals and the occurrence of an evidence of infection is assessed after a period of at least 7 to 10 days, optionally after a period of 21 days after inoculation with the composition to be tested in case the inoculated animal is an adult mouse, after a period of 14 days after inoculation with the composition to be tested in case the inoculated animal is a suckling mouse, and after a period of at least 42 days after inoculation with the composition to be tested in case the inoculated animal is a guinea pig.

The inoculation of the composition to be tested may be carried out intracerebrally and/or intraperitoneally.

The step of determining the presence or absence of extraneous agents in the composition may be carried out in accordance with regulatory requirements, preferfably in accordance with the requirements of the European Pharmacopoeia, 2005, chapter 2.6.16.

The composition to be tested may be a sample of a cell culture from which the active agent is produced, or a product derived from said cell culture.

Furthermore, it is alternatively possible that the composition to be tested is a seed virus, or a composition containing a seed virus, respectively. A seed virus within the meaning of the present invention is a virus that is intended to be used for the production of an antigen or a vaccine. For instance, a seed virus may be genetically altered to render it safe and able to grow in cell culture or in eggs.

The composition may be a pharmaceutical composition, preferably a vaccine preparation or an intermediate product thereof.

The active agent may be an antigen, preferably an inactivated or attenuated virus, more preferably a viral antigen such as a split virus antigen, a subunit virus antigen or a virosome, or the active agent comprises at least one component of a virus or a virus particle, preferably the active agent is an influenza virus particle.

The active agent may be an antigen encoded by a polynucleotide sequence.

The antibody and the active agent may not be derived from using the same polynucleotide construct.

The polynucleotide constructs may differ in at least one structural and/or functional element, preferably differ with regard to the polypeptide they encode.

The antibody may be used for testing viruses as extraneous agents, for example viruses selected from the group consisting of *Pneumovirinae,* such as the *Pneumovirus* genus, including respiratory syncytial virus (RSV); *Morbilliviruses* of the *Paramyxoviridae* family, such as measles virus; *Enteroviruses* of the *Picomaviridae* family, such as *Coxsackie* viruses, for instance coxsackie B5, echo viruses, enteroviruses group A-D, and rhinoviruses; mammalian *Reoviridae,* in particular orthoreoviruses (e.g. mammalian reoviruses such as reovirus 1, 2, and 3) and rotaviruses; members of the *Retroviridae,* for instance the *Orthoretrovirinae,* such as the retroviruses, *Metapneumoviruses* of the *Paramyxoviridae* family, such as human metapneumovirus (HMPV), or parainfluenza virus type 1, 2, 3, and 4; *Rubulaviruses* of the *Paramyxoviridae* family, such as mumps virus; *Togaviridae,* such as *Rubellavirus; Coronaviridae,* such as the SARS coronavirus and other human coronaviruses such as coronavirus OC43, 229E, NL63, and HKU1; *Rhinoviruses* of the *Picomaviridae* family, such as M-strains of Rhino virus; Varicella Zoster virus (VZV), also known as human herpes virus 2 (HHV3); *Polyomaviridae,* such as the SV-40 polyomavirus, the BK polyomavirus and the JC polyomavirus; Porcine circoviruses; Porcine picornaviruses, such as swine vesicular disease virus (SVDV) and Teschen-Talfan virus; members of the *Parvoviridae,* such as canine parvovirus (CPV), bocaviruses or porcine parvoviruses; Parainfluenza viruses (PIV); members of the *Orthomyxoviridae,* including influenza virus type A and B; members of the *Paramyxoviridae paramyxovirinae,* including PIV-I, PIV-2 and PIV-3; the *Herpesviridae,* such as herpes simplex virus 1 and 2, human herpes simplex virus type 6, 7 or 8, cytomegalovirus and Epstein Barr virus; the *Adenoviridae,* such as the adenoviruses, including human, simian and avian adenovirus, such as avian adenovirus 1; avian circoviruses; avian *Reoviridae,* in particular orthoreoviruses, such as avian reoviruses; members of the *Papillomaviridae,* including human papilloma virus; members of the *Flaviviridae,* such as the West Nile virus; and *Bimaviridae,* such as infectious bursal disease virus (also known as gumboro virus), and/or wherein the antibody is used for testing bacteria as extraneous agents, for example *Chlamydia* bacteria, including *C. trachomatis, C. pneumoniae* and *C. psittaci; and Mycoplasma.*

The polynucleotide construct may comprise a HA (hemagglutinin) and/or NA (neuraminidase) coding sequence.

In a preferred embodiment, the polynucleotide construct comprises any of these HA and/or NA coding sequences, either alone or in combination with each other. It is also possible that the polynucleotide construct contains only parts of these sequences. Preferably, the polynucleotide construct contains the complete sequence or a part of the sequence coding for H1, H2, H3, H5, H6, H7, N1, N2, N3 or N7, either alone or in combination, preferably for H5 alone. In a further preferred embodiment, the polynucleotide construct comprises sequences or part of the sequences coding for H1N1, H2N2, H3N2, H6N1, H7N3 or H7N7, preferably the sequences or part of the sequences coding for H5N1.

The polynucleotide construct may comprise the sequence encoding at least a part of the active agent which is codon optimized, in particular by codon optimization to the subject used for immunization with the polynucleotide construct.

The active agent may comprise an influenza antigen and the polynucleotide construct may comprise a sequence having at least 90 %, preferably at least 95%, and more preferably at least 98 % sequence identity to the nucleic acid shown in SEQ ID NO: 1 or 2.

The active agent may comprise an influenza antigen and the polynucleotide construct comprises a sequence as depicted in SEQ ID NO: 1 or 2.

### Detailed description of the invention

The present invention is now described in more detail by preferred embodiments and examples, which are presented for illustrative purpose.

The present invention provides an efficient, fast and reliable method for the detection of contaminations in compositions by using antibodies that specifically bind to the active agents being present in the composition, with the proviso that said antibody had been raised against an expression product of a polynucleotide construct comprising a sequence encoding at least a part of the active agent. It was found that antibodies, which have been prepared by using recombinant polynucleotide constructs for immunization, are particularly advantageous and useful for the neutralization or inactivation of active agents prior to testing of extraneous or adventitious agents. Such antibodies are especially suitable in testing extraneous agents in compositions containing proteinaceous substances as active agents. As the polynucleotide constructs to be used for preparing the antibodies are synthetically prepared and comprise a polynucleotide which encodes at least a part of an amino acid sequence present in the given active agent, the polynucleotide constructs encode homologous sequences to the target to be neutralized or inactivated, but are not contaminated with extraneous or adventitious agents when adding the antibodies to the composition during testing. Contrary thereto, other immunologic extraneous agent testing using antibodies that would be generated by immunization of animals with a given active agent itself might well lead to the formation of antibodies which are 0 for extraneous agents being present in the composition, thereby raising the risk of false negative results. This undesired production of such antibodies in the animals could for instance be induced by contaminants resulting from a cell culture system in which the active agent was produced. However, as a consequence, unlike using polynucleotide construct immunization for antibody production according to the present invention, antibodies which are then specific for extraneous agents would neutralize the extraneous agents during testing, thereby leading to errors in the analysis. The method according to the present invention allows to avoid or at least to reduce the risk of formation of antibodies specific for contaminations. Thus, e.g. false-negative extraneous agent test results can be avoided. Furthermore, the risk of cross reactivity to occur is lowered. Moreover, the polynucleotide constructs can be tested directly in appropriate test systems to show the absence of contaminating extraneous agents, for instance the constructs can be directly tested by PCR for a wide array of potential contaminants or by applying the methods as described in the European Pharmacopoeia, chapter 2.6.16..

In particular, it has been found that by direct immunization of a subject with a polynucleotide construct that encodes, for instance, a viral antigen such as an influenza viral antigen, it is possible to rapidly generate antibodies that specifically bind this influenza viral antigen, with a view that the subsequent test primarily does not respond to the influenza virus itself. Therefore, by using the method according to the present invention, it is no longer necessary to generate the specific antibodies e.g. by immunizing an animal with the wild-type virus or with a virus particle itself obtained during vaccine preparation process, which significantly reduces the time needed for antibody-generation to be used for the subsequent testing stage. Additionally, it is not necessary any more to rely on the identification and cultivation of a cross-reactive strain, as the polynucleotide constructs can be prepared as soon as a (potential) pandemic or seasonal influenza virus strain has been identified. This is particularly useful in case of a pandemic or seasonal outbreak of influenza, as there is a need for a fast release of pandemic or seasonal vaccines to the market. By applying the method according to the invention, specific antibodies directed against these influenza antigens can be rapidly generated and used for testing and optional further treatment purposes during the process of influenza vaccine preparations. Therefore, the present invention reduces the vaccine release lead times. As the generated antibodies can also be used for quality tests the vaccines or their intermediate products are subjected to, such as extraneous agent tests, the present invention also leads to an improved quality of the vaccines. In addition, by applying the teaching of the present invention, it might be possible to further use an expensive production batch after extraneous agents have been detected by subjecting this batch to a special treatment for inactivating or removing the detected adventitious agent.

All in all, by applying the method according to the invention, an improved quality and safety of the tested compositions and an expenditure of adventitious agent testing can be realized. As a consequence, the release lead times of the tested compositions can be reduced.

Within the meaning of the present invention, the term "extraneous agent" or "adventitious agent" relates to contaminations that might be present in the composition to be tested. An adventitious or extraneous agent is an agent that is not intended to be included in a composition and can adversely influence the properties of a product containing the compositions. For example if the composition constitutes a pharmaceutical preparation or shall be used therefor, an adventitious agent can e.g. be an infectious agent (pathogen), namely an agent capable of infecting a human or animal. Such an infectious agent can be a microorganism, e.g. bacteria, fungi, algae, a virus or parts thereof. The viruses are often also able to grow in systems such as cell cultures that are used for the production of biologicals. Furthermore, the composition can also be a contaminated with host cell DNA.

Typical cell lines used in the production of biologicals, in particular for producing viral particles, are mammalian cell lines including MDCK, CHO, BHK, Vero, MRC-5, PER.C6, WI-38 and the like. Examples of infectious viruses and bacteria that might undeliberately infect such cells, and thus representing potential extraneous or adventitious agents to be tested according to the invention, include for example viruses selected from the group consisting of *Pneumovirinae,* such as the *Pneumovirus* genus, including respiratory syncytial virus (RSV); *Morbilliviruses* of the *Paramyxoviridae* family, such as measles virus; *Enteroviruses* of the *Picomaviridae* family, such as *Coxsackie* viruses, for instance coxsackie B5, echo viruses, enteroviruses group A-D, and rhinoviruses; mammalian *Reoviridae,* in particular orthoreoviruses (e.g. mammalian reoviruses such as reovirus 1, 2, and 3) and rotaviruses; members of the *Retroviridae,* for instance the *Orthoretrovirinae,* such as the retroviruses, *Metapneumoviruses* of the *Paramyxoviridae* family, such as human metapneumovirus (HMPV), or parainfluenza virus type 1, 2, 3, and 4; *Rubulaviruses* of the *Paramyxoviridae* family, such as mumps virus; *Togaviridae,* such as *Rubellavirus; Coronaviridae,* such as the SARS. coronavirus and other human coronaviruses such as coronavirus OC43, 229E, NL63, and HKU1; *Rhinoviruses* of the *Picomaviridae* family, such as M-strains of Rhino virus; Varicella Zoster virus (VZV), also known as human herpes virus 2 (HHV3); *Polyomaviridae*, such as the SV-40 polyomavirus, the BK polyomavirus and the JC polyomavirus; Porcine circoviruses; Porcine picornaviruses, such as swine vesicular disease virus (SVDV) and Teschen-Talfan virus; members of the *Parvoviridae,* such as canine parvovirus (CPV), bocaviruses or porcine parvoviruses; Parainfluenza viruses (PIV); members of the *Orthomyxoviridae,* including influenza virus type A and B; members of the *Paramyxoviridae paramyxovirinae,* including PIV-1, PIV-2 and PIV-3; the *Herpesviridae,* such as herpes simplex virus 1 and 2, human herpes simplex virus type 6, 7 or 8, cytomegalovirus and Epstein Barr virus; the *Adenoviridae,* such as the adenoviruses, including human, simian and avian adenovirus, such as avian adenovirus 1; avian circoviruses; avian *Reoviridae,* in particular orthoreoviruses, such as avian reoviruses; members of the *Papillomaviridae,* including human papilloma virus; members of the *Flaviviridae,* such as the West Nile virus; and *Bimaviridae,* such as infectious bursal disease virus (also known as gumboro virus); and bacteria, such as *Chlamydia* bacteria, including *C*. *trachomatis, C. pneumoniae* and *C*. *psittaci; and Mycoplasma*.

By applying the method according to the invention, the presence of extraneous or adventitious agents, respectively, in a composition can be tested in an efficient, expeditious, and reliable manner. Such a composition to be tested may be any composition that shall fulfill certain quality requirements or any upstream samples thereof. Such compositions may be used in test kits e.g. for detecting diseases or infectious agents in any body liquids or samples of human or animals. The method according to the invention is also suitable for testing compositions used for any laboratory use such as analytical or preparative purpose. Further compositions to be tested within the meaning of the present invention can for instance be samples of a cell culture, from which the active agent is produced, or isolation- or purification products or any intermediate products thereof. The samples can be taken at any process step. The composition can also be a product derived from this cell culture. Again, this product can be a product being derived from the cell culture at any stage, which means that the product can either be a precursor of the final product or the final product itself, or any product in between. Furthermore, it is alternatively possible that the composition to be tested is a seed virus, or a composition containing a seed virus, respectively. A seed virus within the meaning of the present invention is a virus that is intended to be used for the production of an antigen or a vaccine.
Other compositions are pharmaceutical compositions. Preferably the composition is a pharmaceutical composition. A pharmaceutical composition can be used in, or on, the body to prevent, diagnose, alleviate, treat or cure a disease in humans or animals. Preferably, such a pharmaceutical composition is a vaccine preparation or an intermediate product thereof. In case the pharmaceutical composition is a vaccine preparation, it is also possible to test certain samples of the vaccine batches. Other compositions to be tested are for example any sample from a process stage eventually leading to a pharmaceutical or vaccine product, for example a sample from cell cultures from which the active agent is derived, or any intermediate product.

As contaminations with an adventitious agent can occur, or can be suitably tested, any time during the manufacturing process, the method according to the invention may be performed at any stage during the manufacture of said composition.

The composition to be tested comprises at least one active agent. An active agent within the meaning of the present invention denotes any chemical or biological material or compound which is the active principle in the composition. In case of a pharmaceutical composition, the active agent may be a drug compound, such as a biopharmaceutical drug, and especially an expressed polypeptide. Preferably the active agent is an antigen, preferably the antigen is an inactivated or attenuated virus, and more preferably the antigen is a viral antigen. Examples of these viral antigens are for instance virus particles such as partially disrupted virus particles such as split virus antigens, purified envelope antigens such as subunit virus antigens or virosomes. A virosome is a unilamellar phospholipid bilayer vesicle with a suitable mean diameter, for example in the range of from about 70 nm to about 150 nm. Essentially, virosomes represent reconstituted empty virus envelopes, devoid of the nucleocapsid including the genetic material of the source virus. Virosomes are not able to replicate but are pure fusion-active vesicles that contain functional viral envelope glycoproteins such as influenza virus hemagglutinin (HA) and neuraminidase (NA) intercalated in the phospholipid bilayer membrane. Also preferred, the active agent comprises at least one component of a virus or a virus particle, preferably the active agent is an influenza virus particle.

Since the method according to the present invention is especially useful in the field of producing pandemic vaccines, according to a preferred embodiment the active agent is an antigen or vaccine component derived from an influenza virus particle, which can be present in influenza vaccines. These influenza vaccines can be based on any suitable influenza strain(s). Influenza vaccines typically include antigens from at least one strain of influenza A, B and C virus, preferably from at least one strain of influenza A or B. The recommended strains for vaccines can change from season to season. It may also be possible that the vaccine is based on more than one suitable influenza strain. For instance, the influenza vaccine can include two influenza A strains and one influenza B strain. It may further be possible that the vaccine can not only be a mono-, but also a bi-, tri- or multivalent vaccine, preferably the vaccine is a trivalent vaccine. The influenza vaccines containing the active agent, preferably the antigen or the vaccine component derived from an influenza virus particle, can be manufactured by any technique that is known to a person skilled in the art. For instance, the vaccines may be manufactured by using polynucleotide constructs encoding the active agent or part of the active agent, or by infecting eggs or cells with live virus preparations. Preferably, the vaccines are manufactured by infecting eggs or cells with live virus preparations. In case the manufacture of vaccines is cell-based, cells that are capable of hosting growing virus, e.g. mammalian cells such as MDCK (Madin-Darby canine kidney cells), CHO (chinese hamster ovary cells), BHK (baby hamster kidney cells), Vero (cells derived from kidney epithelial cells of the African Greene Monkey), MRC-5 (secondary human lung fibroblasts), PER.C6 (cells derived from human embryonic retinal cells), WI-38 (cells derived from human foetal lung tissue) and the like, are used. Usually, the virus or live virus preparation, respectively, is injected into the cells where it multiplies. The cells' outer walls are then removed, harvested, purified, and inactivated. In a egg-based manufacturing, usually the virus or live virus preparation, respectively, is injected into the egg and accumulates in the fluid surrounding the embryo. The embryo becomes infected so that the virus can multiply. After a certain time period, the virus is harvested, purified, and chemically inactivated. This virus or parts of the virus are used to produce the vaccine.

The term "polynucleotide" as used herein is to be understood as meaning a double-stranded or single-stranded nucleic acid molecule, e.g. a DNA, cDNA, genomic DNA, RNA and/or mRNA molecule or the like. The nucleic acid molecule can be present either as the coding strand or as the complementary strand. The polynucleotide may be of a natural source or produced by gene technological or chemical processes and synthesis methods or may have been derived there from. Preferably, the polynucleotide sequence encodes for an antigen as the active agent.

The antibody generated in step a), which had been raised against an expression product of a polynucleotide construct comprising a sequence encoding at least a part of the active agent, is subsequently contacted with a composition comprising at least one active agent, wherein the antibody binds specifically to the active agent, e.g. by forming an antigen-antibody complex.

In the following, the antibodies used in the method of the present invention are described in detail. They derive from a polynucleotide construct or vector. The term "polynucleotide construct" or "vector", respectively, denotes a molecule that is used for introducing exogenous polynucleotides (or inserts, respectively) into host cells or host organisms. The polynucleotide construct comprises a polynucleotide as described above, preferably the polynucleotide construct or vector is a DNA or RNA sequence, and more preferably a DNA sequence. The polynucleotide construct contains a polynucleotide sequence, or an insert, respectively, which encodes one or more (poly)peptides or proteins. This polynucleotide, or insert, respectively, can be a double-stranded or single-stranded nucleic acid molecule, e.g. a DNA, cDNA, genomic DNA, RNA and/or mRNA molecule or the like. The nucleic acid molecule can be present either as the coding strand or as the complementary strand. The polynucleotide may be of a natural source or produced by gene technological or chemical processes and synthesis methods or may have been derived therefrom. Preferably the polynucleotide sequence codes for (poly)peptides or proteins that represent at least a part of the active agent.

Within the meaning of the present invention, the expression "antibody which had been raised against an expression product of a polynucleotide construct" denotes that the antibody is obtained by immunization of suitable organisms with the polynucleotide construct or by raising antibodies in cells or cell systems. The generated antibodies obtained from this specific immunization, optionally isolated, bind (optional specifically) to (poly)peptides, which are encoded by a sequence being comprised by the polynucleotide construct, wherein the (poly)peptides represent at least a part of the active agents.

Preferably the polynucleotide construct or vector, respectively, used in the method according to the present invention comprises a) a promoter region, b) a polynucleotide or insert, respectively, as disclosed herein, which is operatively linked to the promoter region, and c) optionally, regulatory sequences operatively linked thereto, which may act as transcription, termination and/or polyadenylation signals, enhancer sequences and/or sequences coding for leader signals and/or sequences ensuring an efficient ribosome binding, e.g. a Kozak consensus sequence. Suitable promoters and/or regulatory sequences are well known to a person skilled in the art of molecular biology. In any case the skilled person can find suitable promoters and/or regulatory sequences in the literature, e.g. in relevant scientific journals and gene databases, or can isolate them from any desired organism using standard methods such as described in Sambrook et al., Molecular Cloning: A Laboratory Manual, 3rd edition, Cold Spring Harbor Press, (2000).

Polynucleotide sequences or inserts, respectively, that are suitable for the polynucleotide constructs used in the method according to the invention can be determined by a person skilled in the art based on the sequence coding for the polypeptide forming at least a part of the active agent. In case of viral antigens as active agents, the nucleotide sequences coding for this viral antigens are usually known, for instance in case the viruses or vaccines are prepared by a "reverse genetics" approach (see, for instance, Neumann et al., "Reverse Genetics of Influenza Virus", Minireview, Virology 287, 243-250, 2001, references cited therein as well as later reverse genetics techniques). The whole polynucleotide sequence or parts thereof encoding a suitable polypeptide can be chosen by a person skilled in the art and introduced into the polynucleotide construct. A suitable polypeptide is a polypeptide which is capable of eliciting an effective antibody response in the immunized subject. Suitable polypeptides can be found, for instance, in databases that are known to persons skilled in the art, such as the PubMed database (e.g. http://www.ncbi.nlm.nih.gov/, hftp://www.ncbi.nlm.nih.gov/genomes/FLU/FLU.html, http://www.ncbi.nlm.nih.gov/nuccore/145284465?ordinalpos=1&itool=EntrezSystem2.PEntre z.Sequence.Sequence_ResultsPanel.Sequence_RVDocSum and http://www.ncbi.nlm.nih.gov/nuccore). Preferably, the polypeptide comprises one or more antigenic determinants (epitopes). It is also possible that the polynucleotide construct contains a polypeptide not only coding for one active agent or for one part of an active agent, but also coding for one or more other active agents or parts of other active agents. It is also possible that different polynucleotide constructs containing different polynucleotides or inserts, respectively, are used. However, this might depend on the presence and on the number of different active agents being present in the composition. Thus, if there are different types of active agents present, different types of polynucleotide constructs, each type comprising a sequence encoding a polypeptide representing at least a part of a type of active agent, are made.

Preferably the polynucleotide construct or vector used in the method of the present invention is an expression vector. An expression vector is a vector which is able to control the expression (i.e. the transcription and the translation) of the genes which it contains. Even more preferably the vector is a mammalian plasmid expression vector. An example for such a vector is the plasmid (p) pCMV. Preferably, a vector used within the present invention contains the following features: a) a cytomegalovirus (CMV) promoter, b) a Kozak consensus sequence which is placed in front of the ATG start codon to ensure an efficient ribosome binding and hence the maximum level of protein translation, and c) a transcription termination signal, a poly (A) signal, which is placed at the end of the sequence encoding a (poly)peptide or protein that represents at least a part of the active agent to ensure a proper transcription stop. The coding sequence (i.e. the polynucleotide) may be subcloned into the vector at suitable restriction sites. The resulting plasmid (polypeptide construct or vector, respectively, preferably a DNA construct or vector) may then be used for the transformation of suitable host cells such as bacterial cells, yeast cells, fungus cells, algae cells, plant cells, or insect cells, preferably bacterial cells such as *E.coli* cells. Transformed host cells are cultivated in a suitable medium and then harvested and lysed, and the plasmid is recovered. Suitable protocols for the aforementioned procedures can e.g. be found in Sambrook, et al., Molecular Cloning: A Laboratory Manual, 3rd edition, Cold Spring Harbor Press, (2000), Davis, et al., Basic Methods in Molecular Biology, Elsevier (1986), and Ausubel, et al., Current Protocols in Molecular Biology, Wiley Interscience (1988).

In order to characterize the produced plasmid polynucleotide, in general restriction analysis, gel electrophoresis and further biochemical and molecular biological methods may be used as analytic method. These methods as well as methods used for the generation of the polynucleotide construct described above are well known and many treatises on recombinant polynucleotide methods have been published, including Sambrook, et al., Molecular Cloning: A Laboratory Manual, 3rd edition, Cold Spring Harbor Press, (2000), Davis, et al., Basic Methods in Molecular Biology, Elsevier (1986), and Ausubel, et al., Current Protocols in Molecular Biology, Wiley Interscience (1988).

The transformation of the host cells can be used for the amplification of the expression vector. This amplified expression vector can then, in turn, be used e.g. for immunizing a subject as described below.

In order to generate antibodies or antiserum specifically binding to the active agent being present in the composition to be tested, in a preferred embodiment of the invention a subject is immunized with the polynucleotide construct comprising a polynucleotide or insert, respectively, encoding at least a part of the active agent. This direct immunization of a suitable subject with the polynucleotide construct leads to a more rapid generation of antibodies directed against the expression product, as, for instance, process steps can be avoided that might be necessary in case the immunization is carried out with the active agent, e.g. the (poly)peptide, itself. Such otherwise required process step can for instance be the laborious purification of the (poly)peptide. The time-saving, expeditious method according to the present invention is particularly advantageously in case the active agent is a viral antigen, a virus particle, a virosome or any parts of the aforementioned, because of testing a safe, high-quality vaccine can be provided more rapidly. This is in particular advantageously in case of a pandemic or seasonal outbreak of influenza, as there is a need for a fast release of pandemic or seasonal vaccines to the market. By applying the method according to the invention, specific antibodies directed against, and being homologous to the current pandemic or seasonal strain of these influenza antigens can be rapidly generated and used in the generation of influenza vaccine preparations. Similar situations may apply to other vaccine preparations. Furthermore, as the immunization may be carried out with a synthetic polynucleotide construct the risk of immunizing a suitable animal with contaminations is substantially reduced. As a consequence, the risk of generating antibodies directed against these contaminations can be avoided or at least reduced, therefore avoiding e.g false-negative extraneous agent test results. This will significantly enhance the safety and quality of the tested compositions.

The subject can also be immunized with two or more different types of polynucleotide constructs, each type carrying a different polynucleotide. The subject being immunized is a non-human subject, such as a suitable animal, for example a sheep, a goat, a rabbit, a rat, a mouse, a dog, or a guinea pig, preferably a rabbit, a mouse, a guinea pig or a rat, and more preferably a rabbit. Immunization of the suitable animal may be carried out following well-known standard procedures. In general, the optionally purified polynucleotide construct is preferably introduced into animal tissue by a number of delivery methods, such as injection of the polynucleotide construct in saline, using a standard hypodermic needle, gene gun delivery or pneumatic injection. Additionally, the delivery can be carried out by means of topical applications, or a cytofectin-mediated delivery can be carried out. The immunized animal then produces antibodies being specific for at least parts of, or the whole of, the expressed (poly)peptides or proteins. The antibodies produced are present in the blood of the immunized subject and may be recovered by standard procedures that are well known to skilled persons. It is also possible to prepare serum samples from the collected blood of the immunized subjects. The antibodies can be monoclonal or polyclonal, depending on the nature of the (poly)peptide or protein being expressed. The term "monoclonal antibody" within the meaning of the present invention denotes antibodies that have the same antigenic specificity, i.e. antibodies that are all specific for the same epitope (antigenic determinant). That means, if the (poly)peptide being expressed corresponds to a single epitope, then the antibodies are "monoclonal antibodies" within the terms of the present invention. In case the (poly)peptide or protein being expressed comprises more than one epitope, the specific antibodies being produced by the immunized animal are "polyclonal antibodies" within the terms of the present invention.

After a certain time period, for example up to 100 days and usually up to 70 days or up to 50 days, preferably around 70 days after immunization, the specific antibodies generated by the immunized animals can be obtained according to methods well known to persons skilled in the art. Preferably, the animals are exsanguinated and serum samples containing the antibodies are obtained. In a preferred embodiment, the antibody to be used for step a) is simply represented by serum sample obtained from the immunized animal.

Prior to step b), the active agent is neutralized or inactivated by binding of the antibody. Preferably the binding is specific.
The term "specific binding" within the meaning of the present invention denotes that the antibody according to the present invention exhibits specificity for the active agent being present in the composition to be tested, and, therefore, binds selectively to said active agent but not to the extraneous agents being potentially presented in the composition to be tested. In a preferred embodiment of the present invention, the antibody used in the method according to the present invention exclusively binds to said active agent and not at all to extraneous agents being potentially present. In case the active agent is an antigen as described above, the specific binding of the antibody to the active agent might also be a cross-reaction, which means that the antibody according to the present invention exhibits cross-reactivity. The term "cross-reactivity" within the meaning of the present invention denotes the ability of a particular antibody to react with two or more antigens that possess a common or highly homologous epitope. This could for instance be the case in situations where two or more active agents, or two or more antigens, respectively, are present in the composition to be tested.

A neutralized active agent within the meaning of the present invention is an active agent that interacts with the specific antibody, e.g. that forms a complex with the specific antibody, and is therefore essentially not able to be effective any more. In a preferred embodiment, the neutralized active agent is entirely ineffective. An ineffective active agent within the meaning of the present invention is for instance not able any more to carry out its function, such as its pharmacological function. It might also be that the ineffective active agent is not able any more to cause pathogenic effects when contacted with active agent-sensitive detector cell lines or when administrated to test animals. By each of the aforementioned measures, it is ensured that the adventitious agent test at least primarily does not respond to the active agent itself.

In a preferred embodiment of the present invention, the specific antibody reacts with the viral antigen or the virus particle, or the at least one component of the viral antigen or the virus particle, and, thus, destroys or inhibits its pathogenicity, e.g. its infectivity and/or virulence. Neutralizing potency of the specific antibody optionally may be tested prior to performing step b). Neutralizing tests that are well known to persons skilled in the art can be performed. One example for such a neutralizing test is to mix the obtained specific antibodies or the serum containing the specific antibodies with a reference strain that is cross-reactive to the specific antibodies. The reacted reference strain may then be inoculated on detector cell lines being sensitive to infections with the reference strain, such as Vero, MRC-5 or MDCK cell lines. These detector cell lines are then observed for a suitable period of time, for example for about 14 days, and checked for the presence of pathogenic effects. Another possibility of testing the neutralizing potency of an antibody is to test the infectivity of the neutralized preparation by testing the infectivity in an egg model (this test is described for instance in the European Pharmacopoeia, chapter 2.6.16). A pathogenic effect is an adverse effect on the growth or maintenance of a cell, particularly the effects associated with microbial and/or viral infections. Pathogenic effects include, but are not limited to, cytopathic effects (CPE), cell rupture, inhibition of growth, inhibition of protein synthesis, or apoptosis. A CPE is an observable change in cell structure which may vary with cell types and cause of death, and can be determined according to established knowledge in the art. For example, some of the most common effects of viral infection are morphological changes such as cell rounding and detachment from the substrate, cell lysis, syncytium formation and inclusion body formation. A neutralizing activity is characterized by a reduction of the CPE and haemagglutination inhibition, or by a reduced haemadsorption of red blood cells to infected cells. The neutralizing activity can also be characterized by haemagglutination test on cell supernatants.

However, such neutralization tests are reference tests. This means if the neutralizing potency of an antibody could be shown once for a given developed system, this neutralizing test does not have to be carried out consistently for this system.
If the tested serum or antibody, respectively, is not found to have sufficient neutralizing activity, further optimization could be performed regarding the vector design, species used for immunization, dose and route of administration of the DNA constructs or vectors, respectively, immunization and blood collection schedules, and format of neutralizing potency testing. "Sufficient neutralizing activity" within the meaning of the present invention denotes that the antibody/active agent complex is not able to cause detectable effects when carrying out a suitable neutralization test.

According to the inventive method, in step b) the presence or absence of the extraneous or adventitious agent, respectively, is determined. These tests can be carried out in adult mice, suckling mice and guinea pigs according to compendial requirements, for instance according to the requirements of the European Pharmacopoeia, 2005, chapter 2.6.16. (virus seed lot). For virus propagated in avian tissues, a test of avian viruses is carried out as described in the European Pharmacopoeia, 2005, chapter 2.6.16. (virus seed lot and virus harvest).

Further, the construct can also be used for active immunization of the animal test systems prior to inoculation with the composition to be tested as described in chapter 2.6.16. of the European Pharmacopoeia, 2005 (2.6.16.). This would make prior neutralization of the seed virus no longer needed, without altering the ability of the animal test model to respond to contaminating agents. This will further increase the robustness of the test system, reduce the number of animals used in the framework of testing according to 2.6.16, and significantly reduce the time needed to show compliance with 2.6.16.

The antibody and the active agent may not be derived using the same polynucleotide, preferably DNA, construct. A polynucleotide construct can be subject to different purposes of either generating antibodies against the active agent for adventitious agent testing according to the present invention, or of producing the active agent on a preparative scale, this means that the polynucleotide construct being used for the production of the active agent, e.g. the viral antigen or the virus particle, is not used for the generation of the specific antibodies neutralizing or inactivating this active agent.

The expression "not derived from using the same polynucleotide construct" denotes that the polynucleotide construct differs in at least one structural and/or functional component, e.g. with respect to certain parts of the whole construct or has been prepared in a different system. For instance, it is known to a skilled person, that polynucleotide constructs or vectors, respectively, can differ with respect to the functional elements they contain, depending on what they are intended to be used for. If a vector is, for example, only used for multiplying the polynucleotide or insert, respectively, in a suitable host cell, it should contain at least an origin of replication (ori) that allows for semi-independent replication of the vector and the comprised insert in the host cell. In addition, such vectors may contain additional functional elements, such as a multiple cloning site (MCS) which includes nucleotide overhangs for insertion of an insert, or multiple restriction enzyme consensus sites that allow for the insertion of the polynucleotide. If the transcription of the insert is desired, then the vector should additionally contain a promoter sequence. However, these vectors typically lack functional sequences that are necessary for the expression of the polynucleotide. In case the expression of the polynucleotide is desired with a view to provoke enhanced antibody generation against the expressed polypeptide, the vectors additionally comprise a polyadenylation sequence that creates a polyadenylation tail at the end of the transcribed pre-mRNA that protects the mRNA from exonucleases and ensures transcriptional and translational termination. Furthermore, this polyadenylation tail stabilizes the mRNA production. Additionally, only a minimal length of the untranslated region (UTR) or no UTR at all is favored, as the UTRs contain specific characteristics that may impede transcription or translation. Moreover, these vectors should also comprise a Kozak sequence in the mRNA, which assembles the ribosome for translation of the mRNA.

In case the active agent being present in a composition to be tested is a viral antigen or a virus particle generated by using a polynucleotide construct, therefore, the viral antigen or a virus particle is generated by using a polynucleotide construct that is preferably different from the polynucleotide construct used for the immunization of a suitable animal, i.e. used for the generation of antibodies for step a) that preferably specifically bind to the expression product of the polynucleotide sequence being comprised by the polynucleotide construct used for immunization. For instance, these polynucleotide constructs may differ with regard to the presence of a polyadenylation sequence, an untranslated region or a promoter region, and more preferably with regard to the presence of a polyadenylation sequence.

If otherwise the polynucleotide constructs used for the production of the active agent were not only encoding the sequence of this active agent but also a sequence coding for a contamination, then, upon immunization of a subject with this polynucleotide construct, this contamination encoding sequence beside the active agent coding sequence could be expressed in said subject. As a result, the immunized subject would generate antibodies being specific not only for the active agent, but also for the contaminating (poly)peptide. As a consequence, these contamination-specific antibodies could neutralize the contaminations, thereby leading to false-negative test results.
However, by using different polynucleotide constructs, false negative test results are avoided in an extraneous agent test as no antibodies were generated against this contaminating polypeptide. As described above, the polynucleotide constructs can differ in structural and/or functional elements, depending on what they are intended to be used for. In case the polynucleotide construct is, according to the present invention, used for the generation of specific antibodies, preferably an expression vector is used, as the expression of the polynucleotide encoding at least a part of the active agent in the immunized subject is desired. Alternatively, it is also possible that the polynucleotide constructs differ with regard to the respective polynucleotide they encode: By applying the present invention it is, for instance, not necessary that the expression vector used for the immunization of a subject carries the polynucleotide sequence encoding the whole active agent. For the generation of specific antibodies in an immunized subject it could also be suitable that the expression vector only carries a polynucleotide sequence coding for a part of the active agent, such as coding for one or more conserved regions of the active agent.

Furthermore, in a preferred embodiment of the present invention, the polynucleotide sequence encoding at least a part of the active agent can be codon optimized, in particular codon optimized to the subject used for immunization with the polynucleotide construct (see below). By carrying out the above-outlined modifications - using different structural and/or functional elements, expressing a polynucleotide sequence that encodes e.g. only a part of the active agent and codon optimization of the polynucleotide sequence - the risk of additionally expressing a sequence encoding contaminations is almost non-existent. This is particularly advantageous if the composition to be tested shall meet high quality requirements, e.g. to be used for pharmaceutical compositions such as vaccines. Moreover, the above-mentioned modifications of the polynucleotide might additionally lead to a significantly improved expression rate, thereby resulting in an enhanced antibody production and thus enhanced neutralization capacity.

In a further preferred embodiment, the polynucleotide construct comprised sequence encoding at least a part of the active agent is codon optimized, in particular by codon optimization to the subject used for immunization with the polynucleotide construct. As it is known to a person skilled in the art, each specific amino acid is encoded by a minimum of one codon and a maximum of six codons. Prior research has shown that codon usage in genes encoding the cell's polypeptides is biased among species (Kanaya, S, Y. Yamada, Y. Kudo and T. Ikemura (1999), "Studies of codon usage and tRNA genes at 18 unicellular organisms and quantification of Bacillus subtilis tRNAs: gene expression level and species-specific diversity of codon usage based on multivariate analysis.", Gene 238:143-155). The degeneration of the genetic code offers one skilled in the art among other things the possibility of adapting the polynucleotide sequence to the codon preference of the target host cell, thereby optimizing the expression of the desired antigen-binding polypeptide of the present invention. It is known to a person skilled in the art how to adapt the polynucleotide sequence to the codon preference of the target host cell or organism that is immunized with the polynucleotide construct. For instance, if the immunized organism is a suitable animal such as a rabbit, a sheep, a goal, a rat or a mouse, the polynucleotide sequence may be adapted to the codon preference of the respective animal. There exist some software tools (algorithms) for optimizing each gene design for both organism-specific codon usage and for organism-specific codon pair usage, for example "Protein Translation Engineering^{®} technologies" by CODA genomics.

The antibody is used for neutralizing or inactivating the given active agent, with a subsequent testing for viruses and/or bacteria as extraneous agents. These tested viruses and/or bacteria can for instance be selected from the group consisting of *Pneumovirinae,* such as the *Pneumovirus* genus, including respiratory syncytial virus (RSV); *Morbilliviruses* of the *Paramyxoviridae* family, such as measles virus; *Enteroviruses* of the *Picomaviridae* family, such as *Coxsackie* viruses, for instance coxsackie B5, echo viruses, enteroviruses group A-D, and rhinoviruses; mammalian *Reoviridae,* in particular orthoreoviruses (e.g. mammalian reoviruses such as reovirus 1, 2, and 3) and rotaviruses; members of the *Retroviridae,* for instance the *Orthoretrovirinae,* such as the retroviruses, *Metapneumoviruses* of the *Paramyxoviridae* family, such as human metapneumovirus (HMPV), or parainfluenza virus type 1, 2, 3, and 4; *Rubulaviruses* of the *Paramyxoviridae* family, such as mumps virus; *Togaviridae,* such as *Rubellavirus; Coronaviridae,* such as the SARS coronavirus and other human coronaviruses such as coronavirus OC43, 229E, NL63, and HKU1; *Rhinoviruses* of the *Picomaviridae* family, such as M-strains of Rhino virus; Varicella Zoster virus (VZV), also known as human herpes virus 2 (HHV3); *Polyomaviridae,* such as the SV-40 polyomavirus, the BK polyomavirus and the JC polyomavirus; Porcine circoviruses; Porcine picornaviruses, such as swine vesicular disease virus (SVDV) and Teschen-Talfan virus; members of the *Parvoviridae,* such as canine parvovirus (CPV), bocaviruses or porcine parvoviruses; Parainfluenza viruses (PIV); members of the *Orthomyxoviridae,* including influenza virus type A and B; members of the *Paramyxoviridae paramyxovirinae*, including PIV-I, PIV-2 and PIV-3; the *Herpesviridae,* such as herpes simplex virus 1 and 2, human herpes simplex virus type 6, 7 or 8, cytomegalovirus and Epstein Barr virus; the *Adenoviridae,* such as the adenoviruses, including human, simian and avian adenovirus, such as avian adenovirus 1; avian circoviruses, avian *Reoviridae,* in particular orthoreoviruses, such as avian reoviruses; members of the *Papillomaviridae,* including human papilloma virus; members of the *Flaviviridae,* such as the West Nile virus; and *Bimaviridae,* such as infectious bursal disease virus (also known as gumboro virus); *Chlamydia* bacteria, including *C*. *trachomatis, C. pneumoniae* and *C*. *psittaci, and Mycoplasma.*

In a further preferred embodiment of the method according to the invention, the polypeptide being contained in the polynucleotide construct comprises a hemagglutinin (HA) and/or neuraminidase (NA) coding sequence. Hemagglutinin can for instance be found on the surface of the influenza viruses. It is an antigenic glycoprotein that is responsible for binding the virus to the cell that is being infected. To date, at least 16 different influenza HA antigens are known. These subtypes are named H1 through H16. NA is an enzyme which cleaves the glycosidic linkages of neuraminic acid. To date, at least nine subtypes of influenza neuraminidase are known. These subtypes can be found, for instance, in databases that are known to persons skilled in the art, such as the PubMed database (e.g. http://www.ncbi.nlm.nih.gov/, http://www.ncbi.nlm.nih.gov/genomes/FLU/FLU.html, http://www.ncbi.nim.nih.gov/nuccore and http://www.ncbi.nlm.nih.gov/nuccore/145284465?ordinalpos=1&itool=EntrezSystem2.PEntre z.Sequence.Sequence_ResultsPanel.Sequence_RVDocSum). In a preferred embodiment, the polynucleotide construct comprises any of these HA and/or NA coding sequences, either alone or in combination with each other. It is also possible that the polynucleotide construct contains only parts of these sequences. Preferably, the polynucleotide construct contains the complete sequence or a part of the sequence coding for H1, H2, H3, H5, H6, H7, N1, N2, N3 or N7, either alone or in combination, preferably for H5. In a further preferred embodiment, the polynucleotide construct comprises sequences orpart of the sequences coding for H1N1, H2N2, H3N2, H6N1, H7N3 or H7N7, preferably the sequences or part of the sequences coding for H5N1.

In a further preferred embodiment of the method according to the invention, the active agent comprises an influenza antigen and the polynucleotide construct comprises a polynucleotide sequence having e.g. at least 90 %, preferably e.g. at least 95 %, and most preferably e.g. 100 % sequence identity to the nucleic acid shown in SEQ ID NO: 1 or 2. Such polynucleotide sequences are codon optimized for an efficient expression and thus DNA vector based immunization concept of influenza antigen in a mammalian subject, preferably for either one or both HA and NA coding sequences of influenza, more particularly those related to H5 and N1, respectively. The polynucleotide construct may for instance also comprise polynucleotide sequences which hybridize to a complementary strand of the above mentioned nucleotide sequences (nucleic acid shown in SEQ ID NO: 1 or 2) or are a degenerate of the above mentioned nucleotide sequences. The terms "to hybridize" or "hybridization" describe the process by which a single-stranded polynucleotide enters into base-pairing with a complementary polynucleotide strand. In the context of the present invention the term "hybridization" means a hybridization under conventional hybridization conditions, preferably under stringent conditions, for example as described in Sambrook et al. (2000), Molecular Cloning: A Laboratory Manual, 3rd edition, Cold Spring Harbour Laboratory Press, Cold Spring Harbour, NY. Suitable stringent conditions include salt solutions of about 0.9 molar at temperatures of 35 deg. C to 65 deg. C. Stringent hybridization conditions may comprise the following conditions:

| | |
|---|---|
| Hybridization buffer: | 7% SDS |
| | 250 mM NaCl |
| | 250 mM K-phosphate buffer pH 7.0 |
| | 1 mM EDTA |
| Hybridization temperature: | 58 deg. C to 60 deg. C |
| Hybridization time: | overnight |
| Washing buffer: | (I) 2 x SSC, 0.1% SDS |
| | (II) 0.2 x SSC, 0.1% SDS |
| Washing temperature and time: | each 2 x 30 min at 55 deg. C to 60 deg. |

The above-mentioned polynucleotides having e.g. at least 90 %, preferably e.g. at least 95 %, and most preferably e.g. 100 % sequence identity to the nucleic acid shown in SEQ ID NO: 1 or 2 also comprise fragments, derivatives, analogues or parts of the polynucleotide sequences. The fragments, derivatives, analogues or parts may also be both naturally occurring variations or mutations, wherein these mutations may have occurred naturally or have been introduced e.g. by targeted mutagenesis. Moreover, the variations can further comprise synthetic sequences.

The term "fragments" is to be understood as parts of the polynucleotide sequence that are sufficiently long to encode one of the described polypeptides. The term "derivative" in this context means that the sequences differ from the polynucleotide sequences described above at one or several position(s), but have a high degree of homology to these sequences. Homology here means a sequence identity of at least 40%, particularly an identity of at least 60% or 70%, preferably of at least 80%, 82%, 84%, 86% or 88%, and particularly preferably of at least 90%, 92%, 94%, 96% or 98%. Variations from the nucleotide sequences described above may be caused, for example, by deletion, substitution, insertion or recombination.

In order to determine the percentage of homology (= identity) between two amino acid or nucleotide sequences, the two sequences are aligned, and the amino acids or nucleotides at each position are compared. If one position within the sequences is occupied by the same amino acid or the same nucleotide, then the molecules at this position are homologous (= identical). The percentage of homology between the two sequences is a function of the number of common positions that are identical (i.e. homology = number of identical positions per total number of positions x 100).

The homology is calculated over the total amino acid or nucleotide sequence area. In order to compare different sequences a variety of programs based on different algorithms are available to the person skilled in the art. The algorithms of Needleman and Wunsch or Smith and Waterman provide especially reliable results. For the sequence alignments and comparisons, the programs "PileUp" ( J. Mol. Evolution., 25, 351-360, 1987 , Higgins et al., CABIOS, 5 1989: 151 153) or "Gap" and "BestFit" [Needleman and Wunsch (J. Mol. Biol. 48; 443-453 (1970) and Smith and Waterman (Adv. Appl. Math. 2; 482-489 (1981)], which are enclosed in the GCG Software-Packet [Genetics Computer Group, 575 Science Drive, Madison, Wisconsin, USA 53711 (1991)], can be used. The sequence homology values mentioned above as percentages can be determined by means of the "Gap" program over the total sequence area with the following adjustments: Gap Weight: 50, Length Weight: 3, Average Match: 10.000 und Average Mismatch: 0.000. These adjustments can be used as standard adjustments for sequence homology analyses.

The use of the polynucleotide constructs according to the invention allows providing fast, efficient and reliable tests for compositions containing active agents. By using antibodies that are provided by immunizing a subject with a polynucleotide construct, contaminations that might occur when immunizing the subject with the active agent itself are avoided. As a consequence, the use of the inventive polynucleotide constructs avoids false-negative test results. Additionally, the generation of the antibodies used in the respective testings is expedited. As a consequence, the testing is faster. This can be particularly advantageously if the active agent in the composition to be tested is constituted by, or is derived from a virus particle, as the vaccine release lead times might be reduced significantly. This is particularly important for vaccines produced on the basis of cell culture technology.

In general, by using the polynucleotide construct comprising a sequence encoding at least a part or an active agent to raise an antibody specifically against said active agent, the absence of any activity of the composition indicates that the antibody inactivated or neutralized the active agent and that no further antigens are present in the composition to be tested.

The absence of any activity responsive to a given active agent within the composition to be tested means that the active agent being present in the composition has been neutralized or inactivated by specific binding of the antibody to the active agent. The neutralized active agent interacts with the specific antibody, e.g. forms a complex with the antibody, and is therefore essentially not able to be effective any more, preferably the neutralized active agent is entirely ineffective. An ineffective active agent within the meaning of the present invention is for instance not able to carry out its function any more, such as its pharmacological or immunological function. It might also be that the ineffective active agent is not able to cause pathogenic effects any more when contacted with active agent-sensitive detector cell lines. The neutralizing potency of the antibody may be tested as described above. However, a composition found to be active still contains active agents and, thus, is effective.

The polynucleotide construct, the active agent, the composition to be tested and the extraneous or infectious agent are described above, as well as the immunization of a suitable subject and the neutralization of the active agent. Preferably, the active agent is an antigen, more preferably a viral antigen or a virus particle, or the active agent comprises at least one component of a virus or a virus particle, preferably the active agent is an influenza virus particle. Preferably, the composition to be tested is a sample from a cell culture from which the active agent is produced or a product derived from this cell culture, also preferred the composition to be tested is a pharmaceutical composition, preferably a vaccine preparation or an intermediate product thereof. Also preferred, the composition to be tested is a seed virus, or a composition containing a seed virus, respectively.
Further preferred, the extraneous or infectious agent is a virus as described above.

By using a polynucleotide construct comprising a sequence encoding at least a part of an active agent to raise an antibody specifically against said active agent, for instance (negative or positive) control tests may be carried out. In such a control test, a composition shall be tested particularly with regard to whether the active agent (against which the antibody is directed) is present in the composition or not. For this purpose, the antibody that is provided by immunization of a suitable subject with the polynucleotide construct is contacted with the composition to be tested. In case the tested composition does not show any activity after the addition or the specific antibody, the active agent is present in the composition. Optionally, before contacting the composition to be tested with the specific antibody, the neutralizing potency of the antibody can be tested as described above.

By using a polynucleotide construct comprising a sequence encoding at least a part of an active agent to raise an antibody specifically against said active agent, an extraneous agent test can be carried out. The extraneous agent test is carried out as described above.

The present invention also relates to a process for testing a pharmaceutical composition at any stage of the production thereof, in particular a vaccine, wherein at at least one time point of the production process a method for testing extraneous agents in the composition as described above is carried out. The extraneous agent test can be carried out at any stage of the production or manufacturing process, respectively, of the pharmaceutical composition. Preferably the test can be carried out on the seed lots or on the virus harvests, and more preferably the test can be carried out on the seed lots. Furthermore, it can also be carried out once or repeatedly, e.g. at the beginning and/or at the end of the cell culturing, or in between. The extraneous agent test can also be carried out on the ready-made vaccine preparation, including for example testing one or more samples out of a production batch.
Optionally, a step of treating the pharmaceutical composition, in particular the vaccine or an intermediate product thereof, and/or a cell culture from which the pharmaceutical composition or the vaccine is derived, is carried out, whereby the extraneous agent is removed and/or inactivated. Methods suitable for the removing of the extraneous agent from the respective composition or cell culture are known to person skilled in the art and comprise chemical and/or physical inactivation or removal methods, for instance filtering methods, adsorbtion methods, chemical treatments by e.g. formaldehyde or beta-propiolactone, physical treatments such as heating and/or electromagnetic irradiation (e.g. UV-C treatment, or irradiation by gamma radiation), or the like. A further benefit of the useful methods of the present invention resides in that, once the presence of an extraneous agent has been demonstrated, the extraneous agent removal step can be specifically adapted to remove and/or inactivate said extraneous agent. For instance, if the extraneous agent being present in the composition to be tested is (optionally) identified, a specific removal and/or inactivating method can be used, which is known to particularly remove and/or inactivate said extraneous agent. By removing and/or inactivating said extraneous agent, e.g. discarding the whole composition batch could be avoided, therefore saving time and money.

Furthermore, also disclosed is the use of an antibody which had been raised against an expression product of a polynucleotide construct comprising a sequence encoding at least a part or the active agent, wherein the antibody specifically binds to the active agent, for the purification of said active agent, and wherein the antibody and the active agent are not derived using the same polynucleotide construct. The terms "antibody which had been raised against an expression product of a polynucleotide construct" and "antibody and the active agent are not derived using the same polynucleotide construct are described above. The term "purification" as used herein includes, but is not limited to, affinity purification, which is used to purify proteins by retaining them on a column through their affinity to other proteins such as antibodies (which have been produced as described herein) that have been immobilized on a solid support, e.g. a column, and separation, e.g. the separation of different antigens. Preferably, the active agent is a virus antigen, in particular an influenza antigen. The use of the antibody according to the present invention allows for a fast and highly specific purification of the virus particles originating from or representing the virus strain whose purification is desired, as the antibodies used for the purification are specifically raised against an expression product of this virus strain.

Preferably, the antibody that is used for the purification of an active agent is an antibody as defined above. It is further preferred that the antibody that is used for purification purposes additionally comprises an affinity tag for binding to a solid phase.

Furthermore, disclosed is a polynucleotide comprising a sequence having e.g. at least 90 %, and preferably e.g. at least 95 % sequence identity to the nucleic acid shown in SEQ ID NO: 1 or 2. Most preferably, the polynucleotide has a nucleic acid shown in SEQ ID NO: 1 or 2. With regard to said polynucleotide, reference is made to the description above.

Disclosed is also a polynucleotide construct comprising a polynucleotide comprising a sequence having e.g. at feast 90 % and preferably e.g. at feast 95 % sequence identity to the nucleic acid shown in SEQ ID NO: 1 or 2. Most preferred, the polynucleotide construct comprises a polynucleotide having a sequence as depicted in SEQ ID NO: 1 or 2. With regard to said polypeptide, reference is made to the description above. The polynucleotide construct is also described above.

Also disclosed are prokaryotic or eukaryotic host cells which comprise the polynucleotides as described above or a polynucleotide construct comprising said polynucleotide. Preferably, the host cells are stably or transrently transformed with the above-described polynucleotide constructs. With respect to the transformation procedure it is noted that the transformation can be carried out according to standard protocols. However, reference is made to Sambrook et al. (2000), Molecular Cloning: A Laboratory Manual, 3rd Ed., Cold Spring Harbour Laboratory Press, Cold Spring Harbour, NY. Still another aspect of the present invention is to provide non-human organisms, transgenic animals as well as transgenic microorganisms containing the above-described polynucleotides or the above-described vectors or polynucleotide constructs, respectively.

Preferably, the host cells or the animals or microorganisms express and synthesize the antigen-binding polypeptide.

These host cells may be any prokaryotic or eukaryotic cells, preferably microorganismic ceffs, more preferably bacterial, yeast, fungus and algae cells. Particularly preferred among the microorganismic cells are *Escherichia coli* cells, *Streptomyces* cell, *Pichia pastoris* cells or *Schizosaccharomyces* cells, and most preferred are *Escherichia coli* cells.

The polynucleotide construct as well as the immunization of the suitable subject is described above. The antibodies can be recovered by any standard procedure that is known to skilled persons. The antibodies can for instance be used for neutralizing or purifying the specific antigen.

The polynucleotide construct comprising the sequence encoding at least a part of an active agent may have at least 90 %, preferably at least 95%, more preferably at least 98 % sequence identity to the nucleic acid shown in SEQ ID NO: 1 or 2, or the polynucleotide construct comprising a sequence as depicted in SEQ ID NO: 1 or 2, and is used for the transformation of suitable host cells such as bacterial cells, yeast cells, fungus cells, algae cells, plant cells, or insect cells, preferably bacterial cells such as *E. coli* cells. Transformed host cells are cultivated in a suitable medium and then harvested and lysed, and the polynucleotide construct is recovered. The amplified polynucleotide construct can then be used for the immunization of a subject as described above. This immunized subject, in turn, generates antibodies directed against the expression product of the polynucleotide construct, e.g. in this case against the protein being at least a part of an active agent. This antibodies can then be used for testing extraneous agents in a composition comprising at least one active agent, wherein the active agent is encoded at least partially by the sequence having at least 90 %, preferably at least 95%, more preferably at least 98 % sequence identity to the nucleic acid shown in SEQ ID NO: 1 or 2, or wherein the active agent is encoded at least partially by the sequence as depicted in SEQ ID NO. 1 or 2.
With regard to the method of testing for said extraneous agents in a composition, reference is made to the description above.

The following drawings and examples illustrate the present invention in more detail, which are presented for illustrative purpose.

### Brief description of the drawings

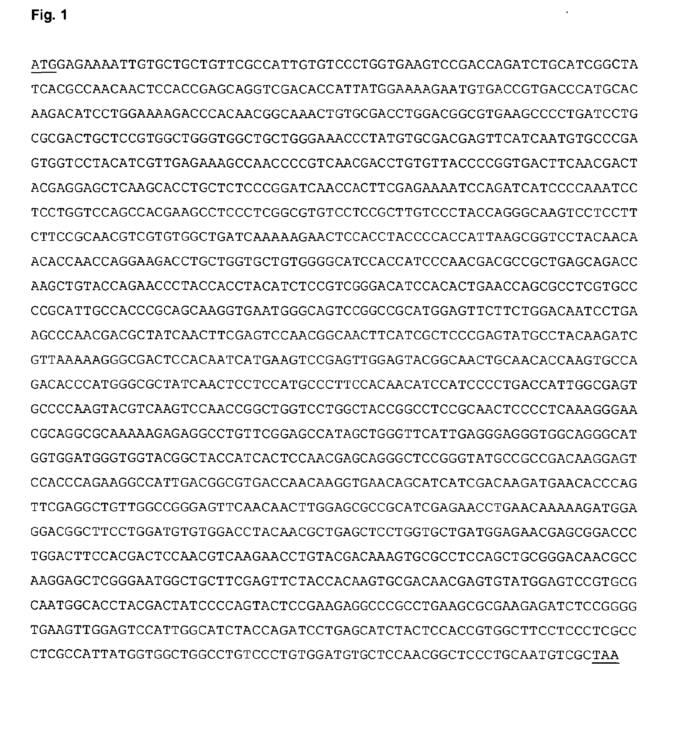

**Fig. 1** shows the nucleotide sequence of the codon optimized hemagglutinin antigen (HA; SEQ ID NO: 1). The first and the last three nucleotides (underlined) represent the start and the stop codon, respectively.

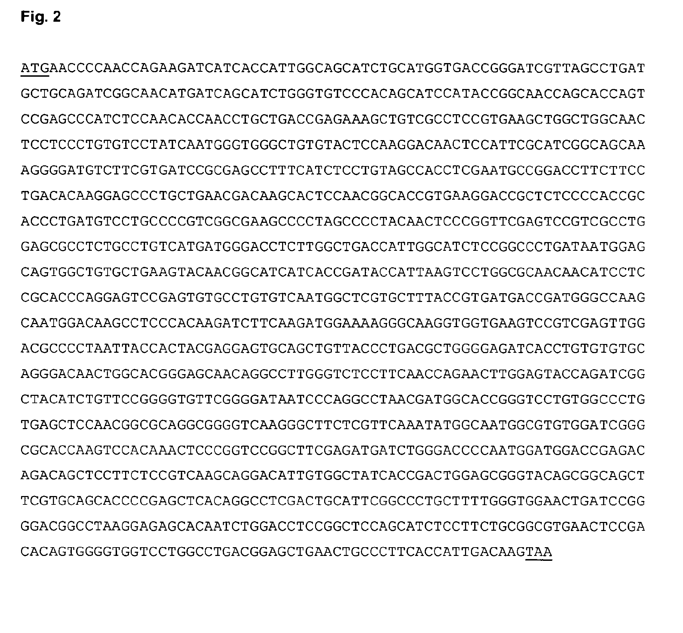

**Fig. 2** shows the nucleotide sequence of the codon optimized neuraminidase antigen (NA; SEQ ID NO: 2). The first and the last three nucleotides (underlined) represent the start and the stop codon, respectively.
**Fig. 3** shows the DNA construct map pCMV-HA, comprising the codon-optimized sequence coding for HA.
**Fig. 4** shows the DNA construct map pCMV-NA, comprising the codon-optimized sequence coding for NA.
**Fig. 5** shows the whole plasmid DNA sequences of pCMV-HA. The restriction enzymes for the plasmid construction are underlined, and the CODA algorithm optimized HA gene sequence is in bold.
**Fig. 6** shows the whole plasmid DNA sequences of pCMV-NA. The restriction enzymes for the plasmid construction are underlined, and the CODA algorithm optimized NA gene sequence is in bold.

### Examples

### 1. Preparation of the polypeptide encoding HA and NA and the DNA construct

DNA vectors have been prepared from the known sequences of the hemagglutinin (HA) and neuraminidase (NA) proteins of the influenza virus A/Viet Nam/1194/2004 (H5N1) (the sequences encoding the HA and the NA, respectively, are disclosed in the PubMed database, see http.//www.ncbi.nlm.nin.gov/, http://www.ncbi.nlm.nih.gov/nuccore/145284463?ordinalpos=1&itool=EntrezSystem2.PEntre z.Sequence.Sequence ResultsPanel.Sequence RVDocSum, and http://www.ncbi.nlm.nih.gov/nuccore/145284406?ordinalpos=1&itool=EntrezSystem2.PEntre z.Sequence.Sequence ResultsPanel.Sequence RVDocSum).

The HA and NA coding sequences were computationally optimized as e.g. described in Roth, D. A. et al., "Translational Engineering and Synthetic Biology", Landes Bioscience, 2007. Specifically, the codon usage and codon-pair usage of the HA and NA coding sequences were optimized according to *Oryctolagus cuniculus.* Within reasonable homology variation range described in the description above, codon optimization could likewise be carried out for other subjects to be immunized by the provided HA and NA coding sequences. The upstream 5' untranslated regions were also optimized to present from unwanted secondary RNA structures that might hinder translation initiation. The CODA algorithm optimized HA and NA genes were assembled and cloned into pCMV vectors, which are commercially available from a whole range of suppliers, such as Clontech Laboratories, Inc.. Both pCMV constructs contained the following features:
- A cytomegalovirus (CMV) promoter driven mammalian expression vector, which was used to produce a high level of RNA transcripts,
- A Kozak consensus sequence placed in front of the ATG start codon to ensure an efficient ribosome binding and hence the maximum level of protein translation,
- A transcription termination signal, the poly (A) signal, was placed at the end of the gene to ensure a proper transcription stop.

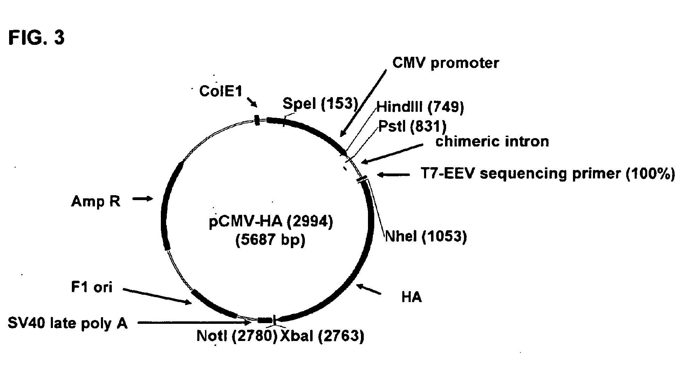

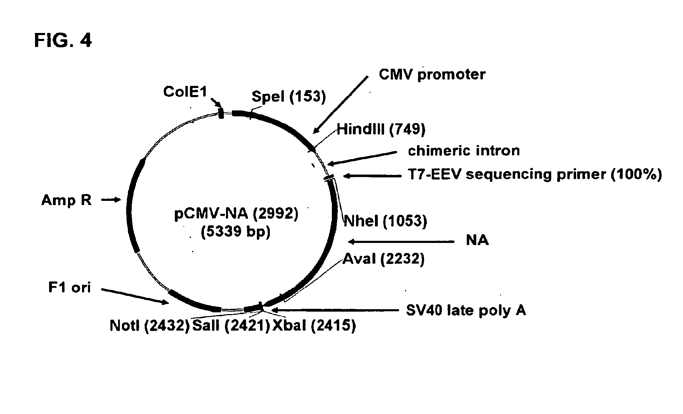

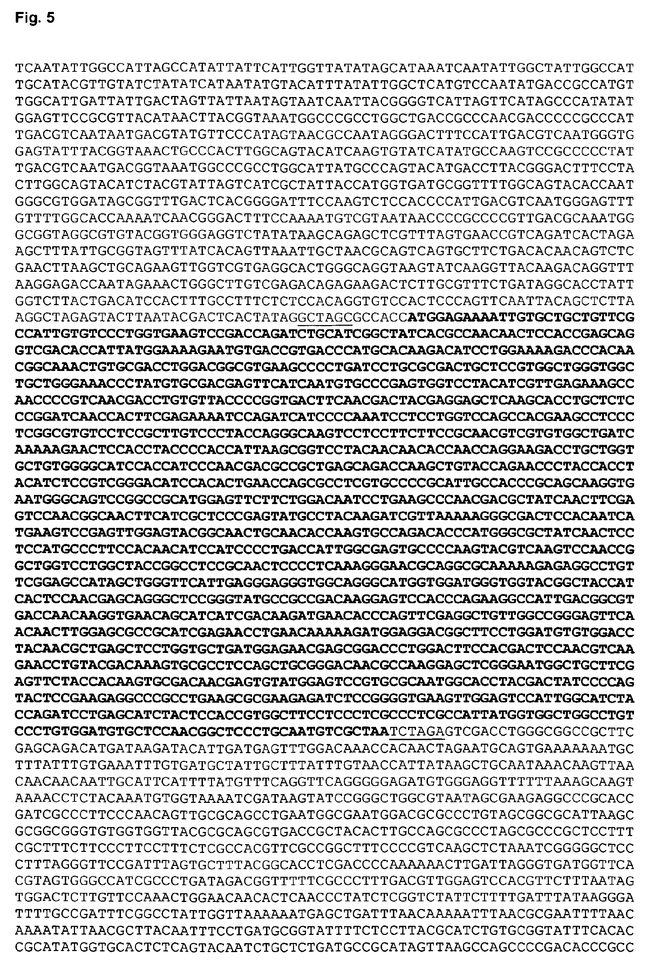

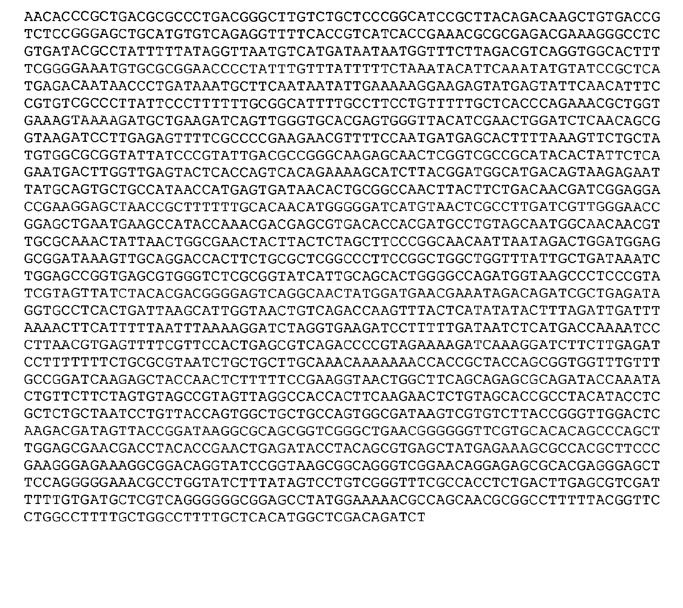

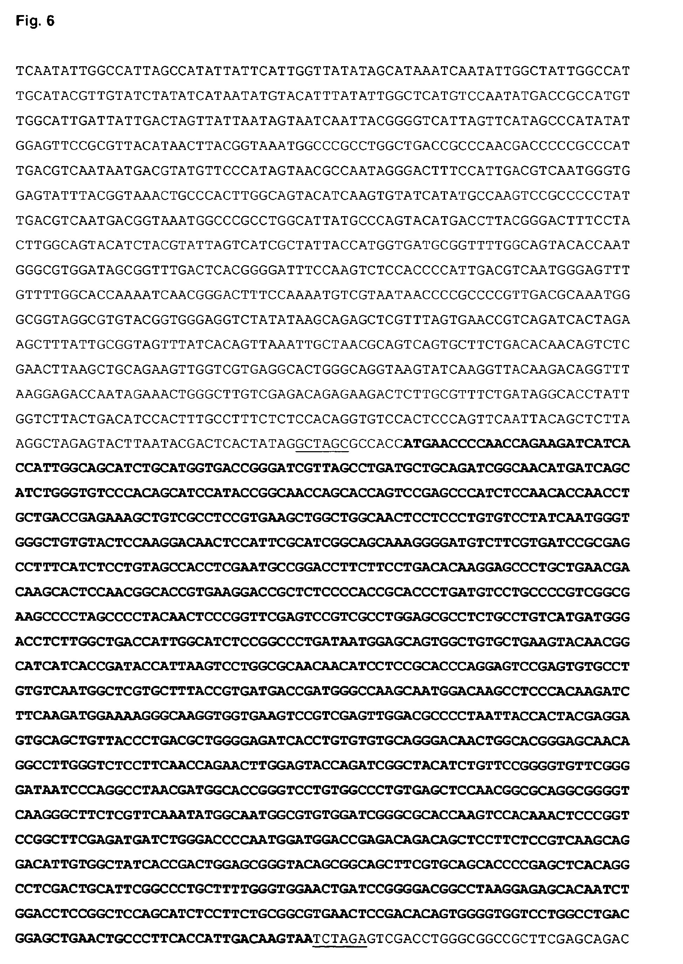

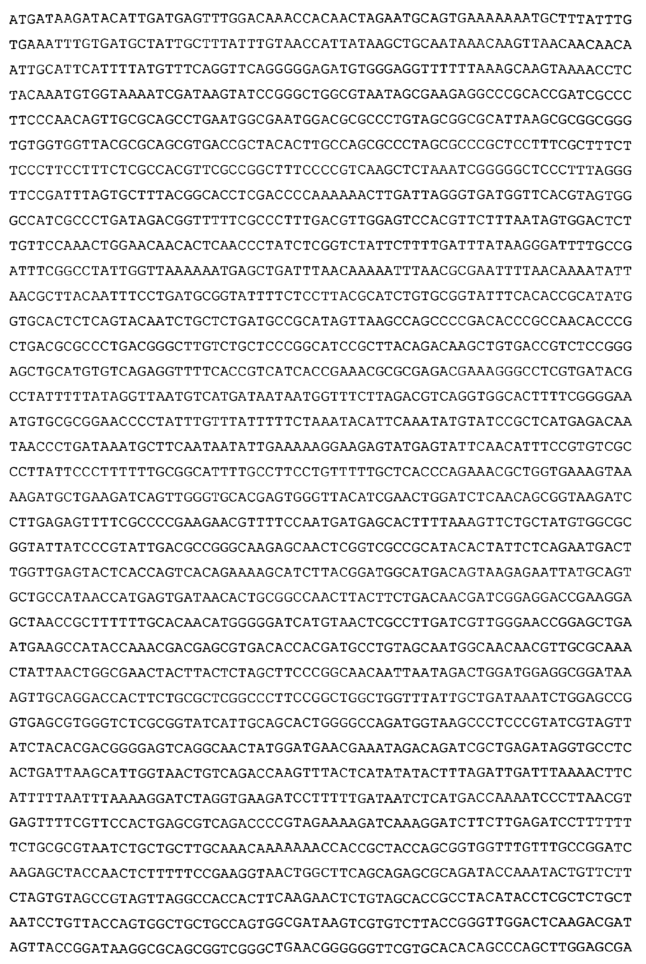

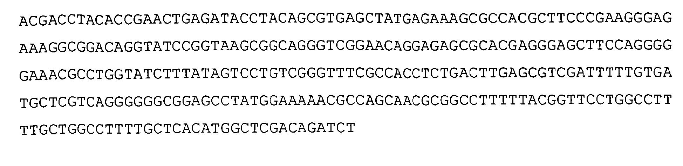

The CODA algorithm optimized HA and NA genes were sub-cloned into the CMV promoter driven vector using restriction enzymes (Nhe I and Xba 1) and named as pCMV-HA and pCMV-NA, respectively. The correct sequences were confirmed by restriction enzyme digestion and by DNA sequencing. The vector maps and DNA sequences are listed in Figures 3-6.

The DNA constructs were separately transfected into *E. coli* cells, from which a Master Cell Bank (MCB) was prepared. The MCB was tested for sterility, bacteriophages, plasmid marker retention, and plasmid identification according to standard methods known to skilled persons.

### 2. Plasmid production

Bulk plasmid production was performed from an *E. coli* culture, followed by subsequent plasmid isolation, purification and characterization. Purified bulk plasmid was tested for DNA integrity, OD 260/280 ratio, agarose gel analysis, restriction analysis, DNA sequence, contaminating proteins, and endotoxins according to standard methods known to skilled persons.

### 3. Immunization of the animals and generation of the antiserum

Subsequently antiserum was generated by immunizing 2 groups of 6 rabbits each. Each rabbit was inoculated with 0.6 ml of DNA material (i.e. the plasmid) on study day 0, 28, and 56. Each dose consisted of 1.0 mg of total DNA. One group of rabbits was immunized with monovalent HA DNA and the other group of rabbits was immunized with a bivalent 1:1 mixture of HA and NA DNA. Pre- and post-immunization blood samples were collected from air animals on day 0, 28, 35, 42 and 56. All animals were terminated by exsanguination on study day 70. All rabbits were healthy, active and free from any clinical signs suggestive of dosing or test article related issues throughout the observation period. All rabbits were healthy and survived throughout the study period and did not show any adverse reaction to antigen.

The obtained blood containing the generated antibodies can be worked up, for example to obtain serum samples containing specific neutralizing antibodies, or to obtain isolated antibody specific for the plasmid/vector expression product.

### 4. Neutralization test

Serum samples can be prepared from the collected blood and testing of the neutralizing potency against Working Seed Virus (WSV) of the pandemic reference strain NIBRG-14 can be carried out. NIBRG-14 is a reassortant A/Viet Nam/1194/2004-like strain used for vaccine manufacture; this strain can be obtained from NIBSC (see http://www.nibsc.ac.uk/ and http://www.nibsc.ac.uk/flu_site/pandemic.html). These serum neutralization tests can be performed as follows:
Two-fold diluted WSV can be mixed with a series of four 3-fold dilutions of the 12 final rabbit bleeds. These serum dilutions can be prepared in a separate flask, and homogenizing and coating of the edges can be performed swerving the solution slightly through the flask. The WSV can be pipetted directly in the serum dilution, and the resulting dilution can be homogenized gently. Then, the dilution can be transferred to a new clean flask and incubated for 2 hours at 37°C by gently swerving on a swerve plate.

One day before inoculation of the cells, 75 cm² flasks of each cell line can be prepared: 6 flaks for neutralized WSV, one for positive and inhibition controls, and one for negative control.

### Positive controls

The positive controls corresponded to the inoculation of about 1000 TCID50 ("Tissue Culture infective Dose", amount of a pathogenic agent that will produce pathological change in 50% of cell cultures) of Human Parainfluenza 3 virus. One positive control can be inoculated at day 0 and used as positive control for the hemadsorption and/or hemagglutination tests at day 14.

### Inhibition controls

Target cells can be inoculated with the sample to be tested previously spiked with about 1000 TCID50 of Human Parainfluenza 3 virus.

### Negative control

For the negative control, dilution medium specific for each cell line can be inoculated per flask. This control can be treated under the same conditions as the sample to be tested.

### Inoculation

The reacted, i.e. potentially neutralized, WSV dilution can then be inoculated on three detector cell lines (Vero cells (obtained from American Type Culture Collection (ATTC) CCL-81; Yasumunra Y. et al., 1962), MRC-5 cells (obtained from ATCC CCL-171; Jacobs J. P. et al., 1970) and MDCK cells (ECACC 84121903; S. H. Darby, 1958)). 3 ml of each solution (dilution medium, sample to be tested) can be inoculated in each flask for the potentially neutralized WSV and controls. After 70 minutes +/- 10 minutes at 37°C +/- 2°C, inoculum can be removed. The survival medium can be then added to obtain a final volume of 20 mf. The culture flasks can be placed at 37°C +/- 2°C in the presence of 5+/-0.5 % CO₂.

The inoculated cells can be regularly observed for 14 days under inverted microscope and checked for the presence of cytopathic effect (CPE) and hemagglutinating activity. The neutralizing potency can be detected by observation of CPE, by hemadsorption tests and by hemagglutination tests.

### 4.1 Observation of CPE

Cells can be regularly observed during the test period under inverted microscope.

### 4.2 Hemadsorption tests

The hemadsorption tests can be carried out at the end of the test period (day 14). The monolyer cells can be washed once or twice with PBS buffer. Then, a solution containing 0.4 % of three types of erythrocytes (human, guinea pig Hartley and rooster) prepared in PBS can be added in each well. The hemadsorption is specific of a viral infection and positive when one type of erythrocytes is fixed on cells. After incubation at 5°C +/- 3°C for about 30 minutes, cells can be subjected to a microscopy examination. The plates can then be incubated at 37°C +/- 2°C for about 30 additional minutes before a second observation.

### 4.3 Hemagglutination tests

The hemagglutination tests can be carried out on the supernatants of cell cultures from flasks used for hemadsorption tests at day 14. The supernatant can be recovered and clarified with a low speed centrifugation. Clarified supernatant can then be placed at 6 well plates. Then a solution containing 0.25 % of the three types of erythrocytes (human, guinea pig Hartley and rooster) prepared in PBS can be added in each well. After about 30 minutes at 5°C +/- 3°C, supernatants can be subjected to a microscopy examination. The 6 well plates can then be incubated at 37°C +/- 2°C for about 30 additional minutes before a second observation.

The tested sample is considered free of viral contaminants using specific cell lines, by observation of the absence of viral CPE and absence of specific hemadsorption and/or hemagglutination activity.

Additionally, the 48 interim bleeds can be tested according to the same procedure, except that only one cell line (MDCK) is used. The results can be compared with those obtained using serum prepared after inoculation of rabbits with inactivated reassortant A/Viet Nam/1203/2004-like virus, a strain that is known to induce cross reacting antibodies in ferrets against A/Viet Nam/1194/2004.

### 4.4 Hen embryonated eggs

According to the European Pharmacopoeia 6th Edition, chapter 2.6.16, hemagglutinating tests can be carried out using hen embryonated eggs.

In brief, SPF (specific pathogen free) eggs (origin of eggs: Couvoir de Cerveloup, 400, domaine de Cerveloup 38210 Vourey, France) can be used that were kept at 12°C +/- 3°C upon receipt until they can be incubated. Incubation can take place at 37°C +/- 2°C in an atmosphere at 70 % of humidity.

### Sample preparation

The WSV can be neutralized with rabbit or sheep antiserum. 1 % antibiotics in aqueous solution to avoid bacterial contaminations of the eggs can be added. Then, the samples can be injected into the eggs with adequate syringe needles.

### Pre-incubation of the eggs

At reception and before pre-incubation, eggs can be grossly observed, and damaged eggs can be discarded. Then, eggs can be pre-incubated at 37°C for 9-11 days. At the end of the pre-incubation period, the eggs can be observed under cold light in order to avoid heating and unfertilized eggs or eggs without living embryo were discarded.

### Inoculation

For analysing the sample to be tested (potentially neutralized WSV), 30 eggs can be inoculated after 9-11 days of incubation via the allantoic route, and for testing the controls, 25 eggs can be inoculated:
As negative control, eggs can be inoculated with PBS supplemented with antibiotics (e.g. 10 eggs), with undiluted rabbit antisera alone (e.g. 5 eggs), and with two types of sheep antiserum alone (e.g. 5 eggs per type).
As positive control for the hemagglutination test, Sendai virus can be used (undiluted). The inoculation can be carried out as follows:
After the disinfection of the egg shells, a hole into the shell can be done and 0.5 ml of the samples to be tested can be inoculated in 55 eggs via allantoic route. The holes in the egg shells can be filled, and the eggs can be incubated at 37°C +/- 2°C for 7 days.

At the end of the incubation period, allantoic fluids from living embryos can be collected after observation. In case dead embryos were observed, the fluid of the respective embryos can be stored at < - 70°C and 5°C+/- 3 °C (in case of bacterial contamination) for further investigations.

### Hemagglutination tests

At the end of the incubation period, hemagglutination tests can be carried out as follows:
The fluids can be clarified by centrifugation (2500 g, 10 minutes), and 200 µl of fluids can be dispatched in four 96 well plates (50 µl per well) in order to perform hemagglutination tests. Briefly, in the wells of two 96 well plates, 50 µl of a solution containing 0.5 % of the erythrocytes (guinea pigs, purchased from Charles River Laboratory, France) can be added, and in the other two 96 well plates, 50 µl of a solution containing 0.5 % of the erythrocytes (hen) can be added. Half of the plates can be incubated at 5°C +/- 3°C, the other half at room temperature. After two hours of incubation, the plates can be checked for hemagglutination activities.

The samples to be tested are considered as being free of viral contaminations, in case no specific hemagglutination activity can be observed in collected fluids from inoculated eggs.

### 5. Testing for adventitious/extraneous agents

The neutralizing serum can then be used for adventitious agents testing. These tests can be carried out according to compendial requirements (European Pharmacopoeia chapter 2.6.16). These tests can be carried out in adult mice, suckling mice and guinea pigs according to compendial requirements, for instance according to the requirements of the European Pharmacopoeia, 2005, chapter 2.6.16. (virus seed lot). With regard to the test that can be carried out in suckling mice, it is noted that the suckling mice can also be inoculated with the composition to be tested at 1 - 2 day(s) of age. For virus propagated in avian tissues, a test of avian viruses can be carried out as described in the European Pharmacopoeia, 2005, chapter 2.6.16. (virus seed lot and virus harvest).

### 5.1 Adult mice: CD1 mice

Three groups of e.g. 10 adult CD1 mice (15-20 g, e.g. purchased from Charles River Laboratory) can be acclimatized for at least 48 hours. One group can receive the neutralized serum (30 µl injected intracerebrally (ic), 500 µl injected intraperitoneally (IP)), one group can be kept in case of death occurrence within the observation period and one group can be used as negative control.
The mice can regularly be observed during the test period (once or twice a day).
No virus is present in the sample to be tested, if 80 % of the adult mice from the group that received the neutralized sample survived at the end of the observation period (21 days).

### 5.2 Suckling CD1 mice

30 mice at age 1-2 day(s) (purchased, e.g., from Charles River Laboratory) can be inoculated with the neutralized serum, 10 mice can be used as negative control. Each suckling mouse receives 10 µl lc. and 100 µl lP of the serum to be tested. The mice can regularly be observed during the test period (once or twice a day).
No virus is present in the sample to be tested, if 80 % of the adult mice from the group that received the neutralized sample survived at the end of the observation period (14 days).

### 5.3 Hartley guinea pigs

From nine Guinea pigs (350-450 g, purchased, e.g, from Charles River Laboratory, France), a group of five animals can be injected IP with 5000 µl of the sample to be tested. Four animals can be observed during the test period of 42 days as negative controls.
No virus is present in the sample to be tested, if no sign of viral infection is detected (i.e. death or macroscopic lesion) during the test period.

## Claims

1. A method for testing extraneous agents in a composition comprising at least one active agent, the method comprising:
a) immunizing of a non-human subject with a polynucleotide construct comprising a sequence encoding a polypeptide forming at least a part of the active agent, in order to generate an antibody raised against the expression product of said polynucleotide construct, contacting the thus generated antibody with the composition comprising at least one active agent, wherein the antibody specifically binds to the active agent, and
b) determining the presence or absence of extraneous agents in the composition subsequent to step a) in an animal model,
wherein the active agent is neutralized or inactivated by binding of the antibody prior to step b).

2. The method according to claim 1, wherein the step of determining the presence or absence of extraneous agents in the composition comprises:
a) using a non-human animal that has been inoculated with said polynucleotide construct, and inoculating said non-human animal with the composition to be tested,
b) assessing the percentage of living animals after a certain period of time, wherein in case at least 80% of the inoculated animals survived and did not show evidence of infection during said time period the composition is regarded as not containing extraneous agents, and in case less than 80% of the inoculated animals survived and/or at least one animal showed evidence of infection during said time period the composition is regarded as containing extraneous agents.

3. The method according to claim 1, wherein the step of determining the presence or absence of extraneous agents in the composition comprises:
a) inoculating a non-human animal with the composition to be tested containing neutralized or inactivated active agent,
b) assessing the percentage of living animals after a certain period of time, wherein in case at least 80% of the inoculated animals survived and did not show evidence of infection during said time period the composition is regarded as not containing extraneous agents, and in case less than 80% of the inoculated animals survived and/or at least one animal showed evidence of infection during said time period the composition is regarded as containing extraneous agents.

4. The method according to claim 2 or 3, wherein the non-human animals are selected from the group consisting of adult mice, suckling mice, and guinea pigs, and wherein the percentage of living animals and the occurrence of an evidence of infection is assessed after a period of at least 7 to 10 days, optionally after a period of 21 days after inoculation with the composition to be tested in case the inoculated animal is an adult mouse, after a period of 14 days after inoculation with the composition to be tested in case the inoculated animal is a suckling mouse, and after a period of at least 42 days after inoculation with the composition to be tested in case the inoculated animal is a guinea pig.

5. The method according to any of claims 2 to 4, wherein the inoculation of the composition to be tested is carried out intracerebrally and/or intraperitoneally.

6. The method according to any of the preceding claims, wherein the composition to be tested is a sample of a cell culture from which the active agent is produced, or a product derived from said cell culture, or a seed virus or a composition containing a seed virus, respectively.

7. The method according to any of the preceding claims, wherein the composition is a pharmaceutical composition, preferably a vaccine preparation or an intermediate product of the vaccine preparation.

8. The method according to any of the preceding claims, wherein the active agent is an antigen, preferably a viral antigen, or wherein the active agent comprises at least one component of a virus particle, preferably the active agent is an influenza virus particle.

9. The method according to any of the preceding claims, wherein the antibody is used for testing viruses as extraneous agents.

10. The method according to any of the preceding claims, wherein the polynucleotide construct comprised a HA (hemagglutinin) and/or NA (neuraminidase) coding sequence.

11. The method according to any of the preceding claims, wherein the polynucleotide construct comprising the sequence encoding at least a part of the active agent is codon optimized, in particular by codon optimization to a subject used for immunization with the polynucleotide construct.

12. The method according to any of the preceding claims, wherein the polynucleotide construct comprises a polynucleotide comprising a sequence having at least 90 %, preferably at least 95 %, more preferably at least 98 % sequence identity to the nucleic acid shown in SEQ ID NO: 1 or 2, or the polynucleotide construct comprises a polynucleotide having a sequence as depicted in SEQ ID NO: 1 or 2.

13. The method according to any of the preceding claims, wherein the polynucleotide construct is contained in a non-human organism, a transgenic non-human animal or a microorganism.

14. The method according to any of the preceding claims, wherein the antibody is specific for a polypeptide encoded by a polynucleotide as defined in claim 12.

15. The method according to claim 14, wherein the antibody is produced by a method comprising:
a) providing a polynucleotide construct as defined in claim 12, and
b) immunizing a suitable subject with said polynucleotide construct.

16. The method according to any of the preceding claims, wherein the active agent comprises an influenza antigen and the polynucleotide construct comprises a sequence having at least 90 %, preferably at least 95%, and more preferably at least 98 % sequence identity to the nucleic acid shown in SEQ ID NO: 1 or 2, even more preferably the active agent comprises an influenza antigen and the polynucleotide construct comprises a sequence as depicted in SEQ ID NO: 1 or 2.

17. A process for testing a pharmaceutical composition at any stage of the production thereof, in particular a vaccine, including, at at least one time point of the production process thereof, carrying out a method according to any one of claims 1 to 16; and, optionally, a step of treating the pharmaceutical composition, in particular the vaccine, or an intermediate product of the production process, and/or treating a cell culture from which the pharmaceutical composition or the vaccine is derived, by a treatment capable of removing and/or inactivating an extraneous agent.

## Patentansprüche

1. Verfahren zum Testen von Fremdstoffen in einer Zusammensetzung umfassend mindestens eine Wirksubstanz, das Verfahren umfassend:
a) Immunisieren eines nicht-humanen Subjekts mit einem Polynukleotidkonstrukt umfassend eine Sequenz, die ein Polypeptid codiert, das mindestens einen Teil der Wirksubstanz bildet, um einen Antikörper zu erzeugen, der gegen das Expressionsprodukt dieses Polynukleotidkonstrukts herangezogen ist, Kontaktieren des so erzeugten Antikörpers mit der Zusammensetzung umfassend mindestens eine Wirksubstanz, wobei der Antikörper spezifisch an die Wirksubstanz bindet, und
b) Bestimmen der Anwesenheit oder Abwesenheit von Fremdstoffen in der Zusammensetzung im Anschluss an Schritt a) in einem Tiermodell,
wobei die Wirksubstanz durch Binden des Antikörpers vor Schritt b) neutralisiert oder inaktiviert wird.

2. Verfahren gemäß Anspruch 1, wobei der Schritt des Bestimmens der Anwesenheit oder Abwesenheit von Fremdstoffen in der Zusammensetzung umfasst:
a) Verwenden eines nicht-humanen Tieres, das mit diesem Polynukleotidkonstrukt inokuliert worden ist, und Inokulieren dieses nicht-humanen Tieres mit der zu testenden Zusammensetzung,
b) Feststellen des Prozentanteils der lebenden Tiere nach einer bestimmten Zeitspanne, wobei, falls mindestens 80% der inokulierten Tiere überlebten und keine Anzeichen einer Infektion während dieser Zeitspanne zeigten, die Zusammensetzung als nicht Fremdstoffe enthaltend betrachtet wurde, und falls weniger als 80% der inokulierten Tiere überlebten und/oder mindestens ein Tier Anzeichen einer Infektion während dieser Zeitspanne aufwies, die Zusammensetzung als Fremdstoffe enthaltend betrachtet wurde.

3. Verfahren gemäß Anspruch 1, wobei der Schritt des Bestimmens der Anwesenheit oder Abwesenheit von Fremdstoffen in der Zusammensetzung umfasst:
a) Inokulieren eines nicht-humanen Tieres mit der zu testenden Zusammensetzung enthaltend neutralisierte oder inaktivierte Wirksubstanz,
b) Feststellen des Prozentanteils der lebenden Tiere nach einer bestimmten Zeitspanne, wobei, falls mindestens 80% der inokulierten Tiere überlebten und keine Anzeichen einer Infektion während dieser Zeitspanne zeigten, die Zusammensetzung als nicht Fremdstoffe enthaltend betrachtet wurde, und falls weniger als 80% der inokulierten Tiere überlebten und/oder mindestens ein Tier Anzeichen einer Infektion während dieser Zeitspanne aufwies, die Zusammensetzung als Fremdstoffe enthaltend betrachtet wurde.

4. Verfahren gemäß Anspruch 2 oder 3, wobei die nicht-humanen Tiere ausgewählt sind aus der Gruppe bestehend aus erwachsenen Mäusen, saugenden Mäusen und Mehrschweinchen, und wobei der Prozentanteil der lebenden Tiere und das Auftreten eines Hinweises auf eine Infektion nach einer Zeitspanne von mindestens 7 bis 10 Tagen, optional nach einer Zeitspanne von 21 Tagen, nach Inokulieren mit der zu testenden Zusammensetzung festgestellt wird, falls das inokulierte Tier eine erwachsene Maus ist, nach einer Zeitspanne von 14 Tagen nach Inokulation mit der zu testenden Zusammensetzung, falls das inokulierte Tier eine saugende Maus ist, und nach einer Zeitspanne von mindestens 42 Tagen nach Inokulation mit der zu testenden Zusammensetzung, falls das inokulierte Tier ein Meerschweinchen ist.

5. Verfahren gemäß irgendeinem der Ansprüche 2 bis 4, wobei das Inokulieren der zu testenden Zusammensetzung intrazerebral und/oder intraperitoneal durchgeführt wird.

6. Verfahren gemäß irgendeinem der vorhergehenden Ansprüche, wobei die zu testende Zusammensetzung eine Probe einer Zellkultur ist, von welcher die Wirksubstanz hergestellt wird, oder ein Produkt, das von dieser Zellkultur abgeleitet ist, oder ein Saatvirus bzw. eine Zusammensetzung, die ein Saatvirus enthält.

7. Verfahren gemäß irgendeinem der vorhergehenden Ansprüche, wobei die Zusammensetzung eine pharmazeutische Zusammensetzung ist, bevorzugt eine Vakzin-Zubereitung oder ein Zwischenprodukt dieser Vakzin-Zubereitung.

8. Verfahren gemäß irgendeinem der vorhergehenden Ansprüche, wobei die Wirksubstanz ein Antigen ist, bevorzugt ein virales Antigen, oder wobei die Wirksubstanz mindestens einen Bestandteil eines Viruspartikels enthält, bevorzugt ist die Wirksubstanz ein Influenza-Viruspartikel.

9. Verfahren gemäß irgendeinem der vorhergehenden Ansprüche, wobei der Antikörper verwendet wird, um Viren als Fremdstoffe zu testen.

10. Verfahren gemäß irgendeinem der vorhergehenden Ansprüche, wobei das Polynukleotidkonstrukt eine HA (Hämagglutinin)- und/oder NA (Neuraminidase)- codierende Sequenz umfasst.

11. Verfahren gemäß irgendeinem der vorhergehenden Ansprüche, wobei das Polynukleotidkonstrukt umfassend die Sequenz, die mindestens einen Teil der Wirksubstanz codiert, Codon-optimiert ist, insbesondere durch Codon-Optimierung auf ein Subjekt, das zum Immunisieren mit dem Polynukleotidkonstrukt verwendet wird.

12. Verfahren gemäß irgendeinem der vorhergehenden Ansprüche, wobei das Polynukleotidkonstrukt ein Polynukleotid, umfassend eine Sequenz mit mindestens 90%, bevorzugt mindestens 95%, mehr bevorzugt mindestens 98% Sequenzidentität mit der in SEQ ID NO: 1 oder 2 gezeigten Nucleinsäure, umfasst, oder das Polynukleotidkonstrukt ein Polynukleotid mit einer Sequenz wie in SEQ ID NO: 1 oder 2 dargestellt, umfasst.

13. Verfahren gemäß irgendeinem der vorhergehenden Ansprüche, wobei das Polynukleotidkonstrukt in einem nicht-humanen Organismus enthalten ist, einem transgenen nicht-humanen Tier oder einem Mikroorganismus.

14. Verfahren gemäß irgendeinem der vorhergehenden Ansprüche, wobei der Antikörper spezifisch ist für ein Polypeptid, codiert von einem Polynukleotid wie in Anspruch 12 definiert.

15. Verfahren gemäß Anspruch 14, wobei der Antikörper hergestellt wird durch ein Verfahren umfassend:
a) Bereitstellen eines Polynukleotidkonstrukts wie in Anspruch 12 definiert, und
b) Immunisieren eines geeigneten Subjekts mit diesem Polynukleotidkonstrukt.

16. Verfahren gemäß irgendeinem der vorhergehenden Ansprüche, wobei der Wirkstoff ein Influenza-Antigen umfasst und das Polynukleotidkonstrukt eine Sequenz mit mindestens 90%, bevorzugt mindestens 95%, und mehr bevorzugt mindestens 98% Sequenzidentität mit der in SEQ ID NO: 1 oder 2 gezeigten Nukleinsäure aufweist, umfasst, noch mehr bevorzugt umfasst die Wirksubstanz ein Influenza-Antigen, und das Polynukleotidkonstrukt umfasst eine Sequenz wie in SEQ ID NO: 1 oder 2 dargestellt.

17. Ein Verfahren zum Testen einer pharmazeutischen Zusammensetzung in irgendeinem Stadium der Herstellung davon, insbesondere eines Vakzins, umfassend zu mindestens einem Zeitpunkt des Herstellungsverfahrens davon, das Ausführen eines Verfahrens gemäß irgendeinem der Ansprüche 1 bis 16; und, optional, einen Schritt des Behandelns der pharmazeutischen Zusammensetzung, insbesondere des Vakzins, oder eines Zwischenprodukts des Herstellungsverfahrens, und/oder Behandelns einer Zellkultur, von welcher die pharmazeutische Zusammensetzung oder das Vakzin abgeleitet ist, mit einer Behandlung, die in der Lage ist, einen Fremdstoff zu entfernen und/oder zu inaktivieren.

## Revendications

1. Procédé permettant de tester des agents exogènes dans une composition comprenant au moins un agent actif, le procédé comprenant les étapes consistant à :
a) immuniser un sujet non humain avec une construction polynucléotidique comprenant une séquence codant pour un polypeptide formant au moins une partie de l'agent actif afin de générer un anticorps dressé contre le produit d'expression de ladite construction polynucléotidique, mettre en contact l'anticorps ainsi généré avec la composition comprenant au moins un agent actif, dans lequel l'anticorps se lie spécifiquement à l'agent actif, et
b) déterminer la présence ou l'absence d'agents exogènes dans la composition après l'étape a) dans un modèle animal,
dans lequel l'agent actif est neutralisé ou inactivé par liaison de l'anticorps avant l'étape b).

2. Procédé selon la revendication 1, dans lequel l'étape de détermination de la présence ou de l'absence d'agents exogènes dans la composition comprend les étapes consistant à :
a) utiliser un animal non humain qui a été inoculé par ladite construction polynucléotidique et inoculer audit animal non humain la composition à tester,
b) évaluer le pourcentage d'animaux vivants après une certaine période de temps, dans lequel, dans le cas où au moins 80 % des animaux inoculés ont survécu et n'ont pas montré de signe d'infection au cours de ladite période de temps, la composition est considérée comme ne contenant pas d'agents exogènes et, dans le cas où moins de 80 % des animaux inoculés ont survécu et/ou qu'au moins un animal a présenté un signe d'infection au cours de ladite période de temps, la composition est considérée comme contenant des agents exogènes.

3. Procédé selon la revendication 1, dans lequel l'étape de détermination de la présence ou de l'absence d'agents exogènes dans la composition comprend les étapes consistant à :
a) inoculer à un animal non humain la composition à tester contenant un agent actif neutralisé ou inactivé,
b) évaluer le pourcentage d'animaux vivants après une certaine période de temps, dans lequel, dans le cas où au moins 80 % des animaux inoculés ont survécu et n'ont pas présenté de signe d'infection au cours de ladite période de temps, la composition est considérée comme ne contenant pas d'agents exogènes et, dans le cas où moins de 80 % des animaux inoculés ont survécu et/ou qu'au moins un animal a présenté un signe d'infection au cours de ladite période de temps, la composition est considérée comme contenant des agents exogènes.

4. Procédé selon la revendication 2 ou la revendication 3, dans lequel les animaux non humains sont choisis dans le groupe constitué de souris, de souriceaux à la mamelle et de cobayes, et dans lequel le pourcentage d'animaux vivants et l'occurrence d'un signe d'infection sont évalués après une période d'au moins 7 à 10 jours, éventuellement après une période de 21 jours après inoculation de la composition à tester dans le cas où l'animal inoculé est une souris adulte, après une période de 14 jours après inoculation de la composition à tester dans le cas où l'animal inoculé est un souriceau à la mamelle et après une période d'au moins 42 jours après inoculation de la composition à tester dans le cas où l'animal inoculé est un cobaye.

5. Procédé selon l'une quelconque des revendications 2 à 4, dans lequel l'inoculation de la composition à tester est effectuée par voie intracérébrale et/ou par voie intrapéritonéale.

6. Procédé selon l'une quelconque des revendications précédentes, dans lequel la composition à tester est un échantillon d'une culture cellulaire à partir de laquelle l'agent actif est produit ou d'un produit dérivé de ladite culture cellulaire, ou d'un virus souche ou d'une composition contenant un virus souche, respectivement.

7. Procédé selon l'une quelconque des revendications précédentes, dans lequel la composition est une composition pharmaceutique, de préférence une préparation vaccinale ou un produit intermédiaire de la préparation vaccinale.

8. Procédé selon l'une quelconque des revendications précédentes, dans lequel l'agent actif est un antigène, de préférence un antigène viral, ou dans lequel l'agent actif comprend au moins un composant d'une particule virale, de préférence l'agent actif est une particule de virus grippal.

9. Procédé selon l'une quelconque des revendications précédentes, dans lequel l'anticorps est utilisé pour tester des virus en tant qu'agents exogènes.

10. Procédé selon l'une quelconque des revendications précédentes, dans lequel la construction polynucléotidique comprend une séquence de codage de HA (hémagglutinine) et/ou de NA (neuraminidase).

11. Procédé selon l'une quelconque des revendications précédentes, dans lequel la construction polynucléotidique comprenant la séquence codant pour au moins une partie de l'agent actif est optimisée au niveau du codon, en particulier par optimisation du codon sur un sujet utilisé pour l'immunisation par la construction polynucléotidique.

12. Procédé selon l'une quelconque des revendications précédentes, dans lequel la construction polynucléotidique comprend un polynucléotide comprenant une séquence ayant au moins 90 %, de préférence au moins 95 %, mieux encore au moins 98 % d'identité de séquence avec l'acide nucléique représenté dans la SEQ ID n° 1 ou 2, ou la construction polynucléotidique comprend un polynucléotide ayant une séquence telle qu'illustrée dans la SEQ ID n° 1 ou 2.

13. Procédé selon l'une quelconque des revendications précédentes, dans lequel la construction polynucléotidique est contenue dans un organisme non humain, un animal non humain transgénique ou un micro-organisme.

14. Procédé selon l'une quelconque des revendications précédentes, dans lequel l'anticorps est spécifique pour un polypeptide codé par un polynucléotide tel que défini dans la revendication 12.

15. Procédé selon la revendication 14, dans lequel l'anticorps est produit par un procédé comprenant les étapes consistant à :
a) fournir une construction polynucléotidique telle que définie dans la revendication 12 et
b) immuniser un sujet approprié par ladite construction polynucléotidique.

16. Procédé selon l'une quelconque des revendications précédentes, dans lequel l'agent actif comprend un antigène de la grippe et la construction polynucléotidique comprend une séquence ayant au moins 90 %, de préférence au moins 95 %, mieux encore au moins 98 % d'identité de séquence avec l'acide nucléique représenté dans la SEQ ID n° 1 ou 2, bien mieux encore l'agent actif comprend un antigène de la grippe et la construction polynucléotidique comprend une séquence telle qu'illustrée dans la SEQ ID n° 1 ou 2.

17. Procédé permettant de tester une composition pharmaceutique à un stade quelconque de sa production, en particulier un vaccin, comprenant, à au moins un moment de son processus de production, la réalisation d'un procédé selon l'une quelconque des revendications 1 à 16 ; et éventuellement une étape de traitement de la composition pharmaceutique, en particulier du vaccin, ou d'un produit intermédiaire du processus de production, et/ou de traitement d'une culture cellulaire d'où la composition pharmaceutique ou le vaccin est tiré(e), par un traitement capable d'éliminer et/ou d'inactiver un agent exogène.
